# EUROPÄISCHE PATENTSCHRIFT

(11) **EP 3 912 709 B1**
(45) Veröffentlichungstag und Bekanntmachung des Hinweises auf die Patenterteilung: **14.02.2024**
(21) Anmeldenummer: 20175365.4
(22) Anmeldetag: 19.05.2020
(51) Int. Cl.: B01F 23/451, B01F 25/27, B01F 25/314, B01F 33/501

(54) **VORRICHTUNG UND VERFAHREN ZUM MISCHEN VON FLÜSSIGKEITEN**
METHOD AND DEVICE FOR MIXING LIQUIDS
DISPOSITIF ET PROCÉDÉ DE MÉLANGE DES LIQUIDES

(43) Veröffentlichungstag der Anmeldung: 24.11.2021
(73) Patentinhaber: Heraeus Medical GmbH, 61273 Wehrheim (DE)
(72) Erfinder: Vogt, Sebastian, 61273 Wehrheim (DE); Kluge, Thomas, 61273 Wehrheim (DE)
(74) Vertreter: Schultheiss & Sterzel Patentanwälte PartG mbB

(56) Entgegenhaltungen:
- WO-A1-2014/090507
- US-A- 4 655 747
- US-A1- 2003 032 937
- US-A1- 2013 317 423

## Beschreibung

Die Erfindung betrifft eine Vorrichtung zum Vermischen kleiner Volumina zweier Flüssigkeiten, insbesondere zweier niedrigviskoser Flüssigkeiten. Die Erfindung betrifft auch ein Verfahren zum Herstellen einer Flüssigkeitsmischung aus zwei Flüssigkeiten mit einer solchen Vorrichtung.

Gegenstand der Erfindung ist insbesondere eine Vorrichtung zum Vermischen von kleinen Volumina von dünnflüssigen Zweikomponentenklebstoffen, speziell von Weichgewebeklebstoffen. Die Vorrichtung ist für die Vermischung von dünnflüssigen Klebstoffkomponenten bestimmt, die in Zweikomponenten-Kartuschen gelagert sind. Die Zweikomponenten-Kartusche kann dabei Teil der erfindungsgemäßen Vorrichtung sein. Weichgewebeklebstoffe sind in der Medizin seit langem bekannt. Für topische Anwendungen kommen häufig Cyanarcylat-Klebstoffe zur Anwendung. Exemplarisch ist dafür ein Klebstoff gemäß US 3 223 083 A. Für Verklebungen innerer Organe haben sich Zweikomponenten-Klebstoffe auf Basis von wässrigen Proteinen-Lösungen bewährt, die mit wässrigen Dialdehyd-Lösungen vernetzt werden. Ein weitverbreiteter Klebstoff basiert auf humanen Serumalbumin und Glutardialdehyd, wie er beispielsweise in der EP 0 650 512 B1 beschrieben wird. Dieser Klebstoff besteht aus zwei sehr dünnflüssigen Komponenten, die üblicherweise mit Hilfe eines statischen Mischers vermischt werden. Die flüssigen Ausgangskomponenten dieses Klebstoffs reagieren innerhalb von wenigen Sekunden von ungefähr 30 bis 45 Sekunden miteinander zu einem gummielastischen gelblichen bis orangen Feststoff. Das bedeutet, wenn aus medizinischen Gründen das Verkleben unterbrochen werden muss, härtet der Klebstoff innerhalb des statischen Mischers aus. Der ausgehärtete Klebstoff kann anschließend nicht aus dem statischen Mischer herausgepresst werden. Die Klebstoffpackung muss verworfen werden. Nachteilig ist hieran also, dass nach jeder Unterbrechung des Klebvorgangs eine neue Zweikomponenten-Kartusche verwendet werden muss. Ein Mischen der Flüssigkeiten in einem Mischbecher ist wegen der hygienischen Anforderungen und der geringen benötigten Menge ebenfalls ungünstig.

Für die Vermischung von zwei flüssigen Komponenten wurden Mischsystem vorgeschlagen, bei denen beide flüssigen Komponenten ineinanderfließen, siehe hierzu beispielsweise die Dokumente DE 1 491 877 A1, EP 0 037 393 A1 und US 4 109 653 A.

Statische Mischer sind seit Jahrzehnten allgemein bekannt. Exemplarische Mischer sind aus den Druckschriften DE 26 48 086 A1, TW M378779 U1, US 3 861 652 A und WO 2019/052838 A2 bekannt.

Die WO 2014/090507 A1 offenbart eine Vorrichtung zum Austragen zweier flüssiger Komponenten mit einer passiven Mischeinheit gemäß dem Oberbegriff des Anspruchs 1. Aus der US 2003/0032937 A1 ist ein Katheter zum Applizieren einer Mischung bekannt, bei dem eine zweite Flüssigkeit über ein durch Druck zu öffnendes Ventilelement in eine erste Flüssigkeit eingespeist werden kann.

Die Aufgabe der vorliegenden Erfindung besteht also darin, die Nachteile des Stands der Technik zu überwinden. Insbesondere besteht die Aufgabe der Erfindung in der Entwicklung einer kostengünstigen und leicht anwendbaren Vorrichtung zum Herstellen eines schnellhärtenden Flüssigkeitsgemischs aus zwei Flüssigkeiten, die auch nach Unterbrechen des Mischvorgangs und nach Aushärten des Flüssigkeitsgemischs in der Vorrichtung weiter zum Mischen und Herstellen und Austragen des Flüssigkeitsgemischs aus den zwei Flüssigkeiten verwendet werden kann. Die Vorrichtung soll dabei ohne Umbaumaßnahmen weiter einsetzbar sein, um das Flüssigkeitsgemisch herzustellen und abzugeben. Der Aufbau soll kostengünstig und einfach Anwendbar sein. Um eine medizinische Anwendung unter hygienischen Bedingungen zu gewährleisten, soll die Vorrichtung nach der Anwendung kostengünstig und hygienisch zu entsorgen sein. Hierzu ist es vorteilhaft, wenn die Vorrichtung möglichst vollständig verbrannt werden kann. Die Vorrichtung soll vom Anwender möglichst einfach bedient werden können.

Die Aufgabe der Erfindung besteht auch in der Entwicklung einer Vorrichtung zur Vermischung von kleinsten Volumina von reaktiven dünnflüssigen Flüssigkeiten und insbesondere von dünnflüssigen Zweikomponentenweichgewebeklebstoffen. Dabei sollen Volumina im Bereich weniger Mikroliter vermischt werden können. Der Mischer der Vorrichtung soll so beschaffen sein, dass im Hohlraum des Mischers nur kleinste Volumina der Flüssigkeiten beziehungsweise der Klebstoffausgangskomponenten enthalten sind. Es soll nicht zu einem Rückfließen der gemischten flüssigen Komponenten in die zulaufenden flüssigen Komponenten kommen können.

Die Aufgaben der Erfindung werden gelöst durch eine Vorrichtung zum Vermischen kleiner Volumina einer ersten Flüssigkeit und einer zweiten Flüssigkeit, die Vorrichtung aufweisend ein Rohr mit einer Leitungsquerschnittsfläche von maximal 2 mm² oder mit einem Innendurchmesser von maximal 1,5 mm, wobei das Rohr ein offenes Ende hat,
eine Hauptleitung, die mit dem Rohr auf der dem offenen Ende gegenüberliegenden Seite flüssigkeitsdurchlässig verbunden ist,
zumindest einen gummielastischen Körper, der eine Leitungswandung der Hauptleitung zumindest bereichsweise begrenzt oder der die gesamte Hauptleitung begrenzt,
zumindest eine Nebenleitung, die in dem zumindest einen gummielastischen Körper ausgeformt ist, wobei die zumindest eine Nebenleitung sich von zumindest einer Nebenöffnung in der Oberfläche des zumindest einen gummielastischen Körpers bis zur Hauptleitung erstreckt und über zumindest eine Einmündung in dem zumindest einen gummielastischen Körper in die Hauptleitung mündet,
einen Hauptanschluss zum Einleiten einer ersten Flüssigkeit in die Hauptleitung,
zumindest eine Zuleitung zum Einleiten einer zweiten Flüssigkeit in die zumindest eine Nebenleitung, wobei die zumindest eine Zuleitung mit der zumindest einen Nebenöffnung flüssigkeitsdurchlässig verbunden ist,
wobei die zumindest eine Nebenleitung in dem zumindest einen gummielastischen Körper ohne Einwirken einer Kraft verschlossen ist und durch Ausüben eines Drucks auf eine der zumindest einen Nebenleitung zugeführte zweite Flüssigkeit hydraulisch durch eine elastische Verformung des zumindest einen gummielastischen Körpers zu öffnen ist und wobei sich die offene zumindest eine Nebenleitung ohne Ausüben eines Drucks auf die zugeführte zweite Flüssigkeit durch die Rückstellkraft des elastisch verformten zumindest einen gummielastischen Körpers verschließt.

Der zumindest eine gummielastische Körper ist bevorzugt zumindest ein gummielastischer Volumenkörper, in dem die zumindest eine Nebenleitung enthalten ist und besonders bevorzugt auch die Hauptleitung enthalten ist.

Beim hydraulischen Öffnen der zumindest einen Nebenleitung wird eine über die zweite Flüssigkeit wirkende Kraft weitergeleitet und dabei der Innendurchmesser und damit der Leitungsquerschnitt der zumindest einen Nebenleitung geöffnet und geweitet. Durch das Weiten der zumindest einen Nebenleitung kann die zweite Flüssigkeit durch die zumindest eine Nebenleitung in die Hauptleitung strömen.

Die zumindest eine Einmündung der zumindest einen Nebenleitung in die Hauptleitung kann mit einem Lippenventil aufgebaut werden. Das Lippenventil oder die Lippenventile sind dann der gummielastische Körper und die zumindest eine Nebenleitung ist die zumindest eine Durchführung durch das zumindest eine Lippenventil. Es handelt sich hierbei um eine spezielle Ausführung der vorliegenden Erfindung mit sehr kompaktem gummielastischem Körper. Bevorzugt wird jedoch ein voluminöserer gummielastischer Körper verwendet, bei dem die Nebenleitungen in dem gummielastischen Körper wenigstens 1 mm lang sind.

Bevorzug ist der Hauptanschluss auf der dem Rohr gegenüberliegenden Seite mit der Hauptleitung verbunden.

Die Vorrichtung ist vorzugsweise eine medizinische Vorrichtung zum Herstellen eines medizinisch anwendbaren Flüssigkeitsgemischs, insbesondere zum Herstellen eines medizinischen Gewebeklebstoffs.

Dass die zumindest eine Nebenleitung in dem zumindest einen gummielastischen Körper ausgeformt ist, bedeutet, dass der zumindest eine gummielastische Körper die Wandungen der zumindest einen Nebenleitung bildet. Das Gleiche gilt gegebenenfalls analog für die Hauptleitung, sofern diese ebenfalls in dem zumindest einen gummielastischen Körper ausgeformt ist.

Bevorzugt sind die erste Flüssigkeit und die zweite Flüssigkeit niedrigviskos. Besonders bevorzugt sind unter niedrigviskosen Flüssigkeiten solche Flüssigkeiten zu verstehen, die bei Raumtemperatur eine Viskosität von höchstens 10 mPa S (0,01 Pa s) haben, ganz besonders bevorzugt eine Viskosität von höchstens 1 mPa S (0,001 Pa s) haben. Die Viskosität kann hierzu beispielsweise mit einem Viskosimeter gemäß DIN EN ISO 2431:2011 gemessen werden.

Der Mischer der Vorrichtung ist der Bereich der Hauptleitung, in dem die zweite Flüssigkeit in die Hauptleitung über die zumindest eine Einmündung eingespritzt werden kann.

Der zumindest eine gummielastische Körper kann eine zylindrische Hülse sein, wobei die Hauptleitung ein sich axial durch die Hülse erstreckender Durchgang ist und die zumindest eine Nebenleitung sich ausgehend von einer Mantelfläche der zylindrischen Hülse bis zu der Hauptleitung erstrecken. Es kann auch vorgesehen sein, dass der zumindest eine gummielastische Körper zumindest eine zylindrische Hülse ist, wobei jede der zumindest einen Nebenleitung sich axial durch jeweils eine der zumindest einen zylindrischen Hülse erstreckt und jeweils eine der Grundflächen der zumindest einen zylindrischen Hülse eine Wandung der Hauptleitung bildet, wobei bevorzugt die die Grundflächen mehrerer zylindrischen Hülsen einander gegenüberliegend und/oder auf der gleichen Höhe der Hauptleitung angeordnet sind.

Das Rohr kann aus einem Kunststoff bestehen, vorzugsweise aus einem Kunststoff bestehen, der sich nicht mit dem Flüssigkeitsgemisch chemisch verbindet und besonders bevorzugt sich auch nicht mit der ersten Flüssigkeit oder der zweiten Flüssigkeit chemisch verbindet.

Der Innendurchmesser einer Leitung oder des Rohrs ist unter der Annahme zu verstehen, dass die Leitung beziehungsweise das Rohr eine kreisförmige oder näherungsweise kreisförmige innere Leitungsquerschnittsfläche im Inneren hat.

Unter der Querschnittsfläche einer Leitung beziehungsweise einer Leitungsquerschnittsfläche ist die offene Fläche senkrecht zur Fließrichtung in der Leitung zu verstehen, die zum Leiten der Flüssigkeit in der Leitung zur Verfügung steht.

Bevorzugt ist die zumindest eine Einmündung im druckfreien Zustand (also, ohne dass ein erhöhter Druck der zweiten Flüssigkeit auf die inneren Wandungen der Nebenleitung wirkt) verschlossen.

Die zumindest eine Einmündung kann zumindest eine Einspritzöffnung sein.

Der zumindest eine gummielastische Körper kann aus allen bekannten biokompatiblen Elastomeren bestehen. Typische geeignete Elastomere sind EPDM, Silikonkautschuk, Naturkautschuk, Butadien-Acrylnitril-Kautschuk und synthetischer Polyisoprenkautschuk.

Es kann vorgesehen sein, dass der zumindest eine gummielastische Körper ein einzelner gummielastischer Körper ist, in dem die Hauptleitung und die zumindest eine Nebenleitung ausgeformt sind.

Hiermit können die Hauptleitung und die zumindest eine Nebenleitung sowie der Verbindung in einem gummielastischen Volumenkörper erzeugt werden, beispielsweise durch Durchbohren und/oder Durchstechen des gummielastischen Volumenkörpers. Dadurch wird der Aufbau vereinfacht, kompakt und kostengünstig.

Dabei kann vorgesehen sein, dass das Rohr in die Hauptleitung eingesteckt ist, wobei bevorzugt das Rohr die zumindest eine Einmündung der zumindest einen Nebenleitung in die Hauptleitung nicht abdeckt.

Hierdurch kann das Rohr auf einfache Weise flüssigkeitsdicht und druckdicht mit der Hauptleitung verbunden werden. Theoretisch kann auch eine seitliche Durchführung oder es können auch mehrere seitliche Durchführungen in der Wandung des Rohrs vorhanden sein, die über die zumindest eine Einmündung geschoben werden, so dass die zumindest eine Einmündung durch die zumindest eine Durchführung in der Wandung des Rohrs in die Hauptleitung beziehungsweise das Rohr einmündet.

Ferner kann dabei vorgesehen sein, dass der gummielastische Körper zumindest im Bereich der Nebenöffnung von einem Mantel abgedeckt wird, wobei bevorzugt der Mantel den gummielastischen Körper koaxial umschließt und die Hauptleitung entlang oder parallel zur Achse des Mantels in dem gummielastischen Körper verläuft, wobei besonders bevorzugt die zumindest eine Zuleitung als zumindest ein flüssigkeitsdurchlässiger Zwischenraum zwischen dem Mantel und dem gummielastischen Körper ausgeführt ist.

Hierdurch wird ein besonders einfacher Aufbau der Vorrichtung erreicht. Der Mantel kann gemäß der bevorzugten Ausführung als eine umlaufende Hülle ausgeführt sein.

Des Weiteren kann vorgesehen sein, dass das Rohr flüssigkeitsdicht und druckdicht mit der Hauptleitung verbunden ist, der Hauptanschluss flüssigkeitsdicht und vorzugsweise auch druckdicht mit der Hauptleitung verbunden ist und die zweite Zuleitung flüssigkeitsdicht und vorzugsweise auch druckdicht mit der zumindest einen Nebenöffnung verbunden ist.

Hiermit wird sichergestellt, dass bei der Verwendung der Vorrichtung weder die erste und die zweite Flüssigkeiten zur Herstellung des Flüssigkeitsgemischs noch das Flüssigkeitsgemisch aus der Vorrichtung außer über das Rohr austreten kann.

Auch kann vorgesehen sein, dass der Innendurchmesser der Hauptleitung oder der Innendurchmesser der Hauptleitung in einem expandierten Zustand den gleichen Innenquerschnitt wie das Rohr hat, und/oder das Rohr in seinem Inneren eine gerade zylindrische Leitung begrenzt, wobei bevorzugt in dem Rohr keine Vorsprünge oder Vertiefungen sind, besonders bevorzugt das Rohr in seinem Inneren glatt ist.

Dadurch kann sichergestellt werden, dass ein sich durch Verfestigen oder Aushärten des Flüssigkeitsgemischs gebildeter Pfropfen gut aus der zylindrischen Leitung, beziehungsweise aus dem Übergang von der Hauptleitung zum Rohr ausgestoßen werden kann, um anschließend neues flüssiges Flüssigkeitsgemisch applizieren zu können. Damit wird sichergestellt, dass nach einer Unterbrechung mit der gleichen Vorrichtung neues Flüssigkeitsgemisch der ersten und der zweiten Flüssigkeiten erzeugt und ausgetragen werden kann.

Auch kann vorgesehen sein, dass die zumindest eine Einmündung flächenbündig mit der Innenwand der Hauptleitung ist und/oder das Rohr flächenbündig mit der Hauptleitung ist oder das Rohr flächenbündig mit der Hauptleitung im expandierten Zustand ist.

Hiermit wird verhindert, dass sich beim Unterbrechen des Austragens des Flüssigkeitsgemischs der ersten und der zweiten Flüssigkeit ein fester Pfropfen in der Hauptleitung bildet, der sich über Hinterschneidungen, die von der zumindest einen Einmündung verursacht werden, fest und unlösbar in der Hauptleitung verankert. Damit wird sichergestellt, dass nach einer Unterbrechung des Austrags des Flüssigkeitsgemischs der Flüssigkeiten mit der gleichen Vorrichtung neues Flüssigkeitsgemisch erzeugt und ausgetragen werden kann. Die Bündigkeit betrifft insbesondere die zumindest Einmündung im geschlossenen Zustand. Im geöffneten Zustand kann die zumindest eine Einmündung theoretisch auch ein wenig in die Leitung hineinragen oder zurückstehen.

Es kann auch vorgesehen sein, dass die zumindest eine Nebenleitung wenigstens 1 mm lang ist, bevorzugt zumindest 5 mm lang ist, und/oder die Hauptleitung mindestens 3 mm lang ist, bevorzugt zumindest 5 mm lang ist.

Hierdurch kann die zumindest eine Nebenleitung vollständig geschlossen werden und es kann ein ungewollter Rückfluss von Flüssigkeitsgemisch durch die zumindest eine Nebenleitung verhindert werden. Da üblicherweise kein Gegendruck auf dem offenen Ende des Rohrs lastet, während die zweite Flüssigkeit mit einem Druck durch die zumindest eine Nebenleitung geleitet werden muss, wird die in der zumindest einen Nebenleitung enthaltene zweite Flüssigkeit bei einem Zusammenziehen (also einem elastischen Verschließen) der zumindest einen Nebenleitung in Richtung des Rohrs in die Hauptleitung eingepresst, weil sich der sich abbauende Druck in der zumindest einen Nebenleitung nur in Richtung des druckfreien weil offenen Endes des Rohrs abbauen wird.

Durch die Mindestlänge der Hauptleitung bleibt immer nichtvermischte Flüssigkeit in der Hauptleitung, wenn derAuspress- und Mischvorgang unterbrochen wurde. Dadurch kann beim erneuten Auspressen der ersten Flüssigkeit und der zweiten Flüssigkeit die erste Flüssigkeit den im Bereich der zumindest einen Einmündung ausgehärteten Klebstoff aus der Hauptleitung und aus dem sich anschließenden Hohlraum des Rohrs heraus schieben, so dass das ausgehärtete Flüssigkeitsgemisch beziehungsweise der ausgehärtet Klebstoff in Form eines Zylinders aus dem offenen Ende des Rohrs austritt beziehungsweise herausgedrückt werden kann.

Des Weiteren kann vorgesehen sein, dass die Hauptleitung über den Hauptanschluss mit einem Hauptreservoir enthaltend die erste Flüssigkeit verbunden ist und die zumindest eine Zuleitung mit einem Nebenreservoir enthaltend die zweite Flüssigkeit verbunden ist, wobei bevorzugt ein erster Austragskolben in dem Hauptreservoir angeordnet ist, mit dem die erste Flüssigkeit aus dem Hauptreservoir in die Hauptleitung einpressbar ist, und in dem Nebenreservoir ein zweiter Austragskolben angeordnet ist, mit dem die zweite Flüssigkeit aus dem Nebenreservoir in die zumindest eine Zuleitung einpressbar ist, wobei besonders bevorzugt der erste Austragskolben und der zweite Austragskolben miteinander fest verbunden sind.

Hierdurch kann die Vorrichtung unmittelbar zum Herstellen des Flüssigkeitsgemischs aus der ersten Flüssigkeit und der zweiten Flüssigkeit verwendet werden, ohne dass diese zuvor in die Vorrichtung eingespeist werden müssten.

Der Druck zum Öffnen der zumindest einen Nebenleitung wird dann über die zweite Flüssigkeit vermittelt. Der Druck auf die zweite Flüssigkeit kann dazu mit Hilfe des zweiten Austragskolbens erzeugt werden.

Ferner kann vorgesehen sein, dass die erste Flüssigkeit und die zweite Flüssigkeit Ausgangskomponenten eines medizinischen Gewebeklebstoffs sind und die Vorrichtung eine Vorrichtung zum Herstellen eines medizinischen Gewebeklebstoffs ist.

Die Vorrichtung ist solche medizinischen Anwendungen besonders gut geeignet und einsetzbar.

Bevorzugt kann vorgesehen sein, dass das Rohr eine Leitungsquerschnittsfläche von maximal 1 mm² oder einen Innendurchmesser von maximal 1 mm, bevorzugt von maximal 0,5 mm hat.

Hierdurch können sehr kleine Volumenströme des Flüssigkeitsgemischs erzeugt werden, wie sie im medizinischen Bereich zur Herstellung von medizinischen Gewebeklebstoffen Anwendung finden.

Auch kann vorgesehen sein, dass die zumindest eine Einmündung oder die zumindest eine Nebenleitung im geöffneten Zustand einen Durchmesser von maximal 0,5 mm hat, bevorzugt einen Durchmesser von maximal 0,3 mm hat.

Das bedeutet, dass die zumindest eine Nebenleitung oder die zumindest eine Einmündung mit Hilfe der Vorrichtung nicht über die genannten Durchmesser hinaus geweitet werden kann.

Hierdurch wird sichergestellt, dass durch die zumindest eine Nebenleitung nur ein geringer Volumenstrom der zweiten Flüssigkeit der durch die Hauptleitung strömenden ersten Flüssigkeit beigemengt werden kann.

Gemäß einer bevorzugten Weiterbildung der vorliegenden Erfindung kann vorgesehen sein, dass die zumindest eine Nebenleitung zumindest zwei Nebenleitungen sind und die zumindest eine Einmündung zumindest zwei Einmündungen sind, wobei bevorzugt die zumindest zwei Einmündungen auf gegenüberliegenden Seiten der Hauptleitung angeordnet sind, so dass die durch die zumindest zwei Einmündungen in die Hauptleitung eingespritzte zweite Flüssigkeit in der Hauptleitung direkt aufeinandertreffen.

Hierdurch wird eine bessere Durchmischung der durch die zumindest zwei Nebenleitungen eingespritzten zweiten Flüssigkeit mit der in der Hauptleitung geleiteten ersten Flüssigkeit erreicht.

Des Weiteren kann vorgesehen sein, dass die Hauptleitung und die zumindest eine Zuleitung mit einer Zweikomponenten-Kartusche verbunden oder verbindbar sind, wobei besonders bevorzugt die Hauptleitung über den Hauptanschluss mit einer ersten Kammer der Zweikomponenten-Kartusche verbunden oder verbindbar ist und die zumindest eine Zuleitung mit einer zweiten Kammer der Zweikomponenten-Kartusche verbunden oder verbindbar ist, wobei ganz besonders bevorzugt in der ersten Kartusche die erste Flüssigkeit als erste Ausgangskomponente enthalten ist und in der zweiten Kartusche die zweite Flüssigkeit als zweite Ausgangskomponente enthalten ist.

Hierdurch kann die Vorrichtung unmittelbar zum Mischen des Flüssigkeitsgemischs eingesetzt werden.

Bevorzugt kann auch vorgesehen sein, dass die Hauptleitung in dem zumindest einen gummielastischen Körper ausgeformt ist oder in einem weiteren gummielastischen Körper ausgeformt ist, wobei die Hauptleitung in dem zumindest einen gummielastischen Körper oder in dem weiteren gummielastischen Körper ohne Einwirken einer Kraft verschlossen ist und durch Ausüben eines Drucks auf die der Hauptleitung zugeführte erste Flüssigkeit hydraulisch durch eine elastische Verformung des zumindest einen gummielastischen Körpers oder des weiteren gummielastischen Körpers zu öffnen ist und wobei sich die offene Hauptleitung ohne Ausüben eines Drucks auf die zugeführte erste Flüssigkeit durch die Rückstellkraft des elastisch verformten zumindest einen gummielastischen Körpers verschließt.

Hierdurch kann ein Aushärten des Flüssigkeitsgemischs in dem gummielastischen Körper vermieden werden, da das Flüssigkeitsgemisch bei einer ausreichenden Reduzierung des Auspressdrucks auf die erste Flüssigkeit aus der Hauptleitung in das Rohr gedrückt wird, da das Rohr an seiner Vorderseite offen ist, während auf der gegenüberliegenden Seite ein Gegendruck durch die erste Flüssigkeit vorhanden ist und sich die Hauptleitung durch das Zusammenziehen der Hauptleitung aufgrund der elastisch verformten Wandung der Hauptleitung in Richtung des offenen Rohrendes entleeren muss.

Ferner kann vorgesehen sein, dass die zumindest eine Einmündung in den zumindest einen gummielastischen Körper trichterförmig ist und eine Öffnung in den zumindest einen gummielastischen Körper bereitstellt, vorzugsweise auch dann, wenn kein Druck auf den zumindest einen gummielastischen Körper wirkt.

Hierdurch wird sichergestellt, dass sich der Druck der zweiten Flüssigkeit in die zumindest eine trichterförmige Öffnung fortpflanzen kann und so die zumindest eine Nebenleitung durch Druckbeaufschlagung geweitet werden kann. Die trichterförmige zumindest eine Einmündung hilft also dabei, dass die zweite Flüssigkeit aus der zumindest einen Zuleitung gut in die zumindest eine Nebenleitung eindringen kann um die dort mit Hilfe eines Drucks auf die zweite Flüssigkeit hydraulisch zu öffnen.

Wenn die Hauptleitung ebenfalls zumindest bereichsweise in dem zumindest einen gummielastischen Körper ausgeformt ist, kann vorgesehen sein, dass eine Haupteinmündung der Hauptleitung in den gummielastischen Körper trichterförmig ist und eine Öffnung in den zumindest einen gummielastischen Körper bereitstellt, vorzugsweise auch dann, wenn kein Druck auf den zumindest einen gummielastischen Körper wirkt. Dann kann auch die (verschließbare beziehungsweise verschlossene) Hauptleitung analog der zumindest einen Nebenleitung gut geöffnet werden.

Die der vorliegenden Erfindung zugrundeliegenden Aufgaben werden auch gelöst durch ein Verfahren zur Herstellung einer Mischung zweier Flüssigkeiten, das Verfahren aufweisend die folgenden chronologischen Schritte:
A) Bereitstellen einer Zweikomponenten-Kartusche aufweisend ein Hauptreservoir, in dem eine erste Flüssigkeit enthalten ist, und ein Nebenreservoir, in dem eine zweite Flüssigkeit enthalten ist;
B) Auspressen der ersten Flüssigkeit aus dem Hauptreservoir in eine Hauptleitung und Auspressen der zweiten Flüssigkeit aus dem Nebenreservoir in zumindest zwei geschlossene Nebenleitungen, wobei die zumindest zwei Nebenleitungen in zumindest einem gummielastischen Körper ausgeformt sind;
C) Öffnen der zumindest zwei Nebenleitungen durch elastisches Verformen des zumindest einen gummielastischen Körpers aufgrund einer von der zweiten Flüssigkeit ausgeübten und hydraulisch vermittelten Kraft auf die Wandungen der zumindest zwei Nebenleitungen;
D) Einspritzen der zweiten Flüssigkeit in die erste Flüssigkeit in der Hauptleitung durch mehrere einander gegenüberliegende Einmündungen der offenen zumindest zwei Nebenleitungen in die Hauptleitung und dadurch Vermischen der ersten Flüssigkeit mit der zweiten Flüssigkeit, wobei die Ströme der eingespritzten zweiten Flüssigkeit in der Hauptleitung aufeinandertreffen und dadurch das Vermischen der ersten Flüssigkeit mit der zweiten Flüssigkeit gefördert wird; und
E) Auspressen des Flüssigkeitsgemischs durch ein an die Hauptleitung angeschlossenes Rohr.

Bevorzugt wird mit dem Verfahren ein medizinischer Gewebeklebstoff hergestellt.

Das Verfahren wird dabei lediglich zur Herstellung des Flüssigkeitsgemischs beziehungsweise des medizinischen Gewebeklebstoffs angewendet. Eine medizinische Anwendung bei einem Patienten erfolgt nicht innerhalb des Verfahrens.

Das Verfahren beinhaltet keine von der Patentierung ausgeschlossenen Verfahrensschritte zur medizinischen Behandlung eines menschlichen oder tierischen Körpers. Solche Schritte erfolgen allenfalls im Anschluss an das erfindungsgemäße Verfahren.

Es kann vorgesehen sein, dass das Verfahren mit einer erfindungsgemäßen Vorrichtung durchgeführt wird, wobei vorzugsweise das Hauptreservoir mit dem Hauptanschluss und der Hauptleitung flüssigkeitsdurchlässig und druckdicht verbunden ist und das Nebenreservoir mit der zumindest einen Zuleitung und den zumindest zwei Nebenleitungen flüssigkeitsdurchlässig und druckdicht verbunden ist oder nach Schritt A) und vor Schritt B) ein Schritt A2) erfolgt: A2) Verbinden der erfindungsgemäßen Vorrichtung oder der restlichen Vorrichtung mit der Zweikomponenten-Kartusche, wobei das Hauptreservoir mit dem Hauptanschluss und der Hauptleitung flüssigkeitsdurchlässig und druckdicht verbunden wird und das Nebenreservoir mit der zumindest einen Zuleitung und den zumindest zwei Nebenleitungen flüssigkeitsdurchlässig und druckdicht verbunden wird.

Hierdurch profitiert das Verfahren von den erfindungsgemäßen Vorteilen der Vorrichtung.

Die restliche Vorrichtung ist die Vorrichtung ohne die Zweikomponenten-Kartusche. Diese Alternative betrifft also Vorrichtungen, bei denen die Zweikomponenten-Kartusche Teil der Vorrichtung ist.

Des Weiteren kann vorgesehen sein, dass das Auspressen in Schritt B) durch axiales Vortreiben je eines ersten Austragskolbens in dem Hauptreservoir und eines zweiten Austragskolbens in dem Nebenreservoir erfolgt, wobei bevorzugt der erste Austragskolben und der zweite Austragskolben synchron vorgetrieben werden und besonders bevorzugt der erste Austragskolben und der zweiten Austragskolben miteinander fest verbunden sind und in Schritt B) gemeinsam vorgetrieben werden.

Hierdurch können die Flüssigkeiten sehr leicht angetrieben werden und der zum Öffnen der zumindest zwei Nebenleitungen, und gegebenenfalls der Hauptleitung, notwendige Druck kann mit den Austragskolben gut auf die zweite Flüssigkeit, und gegebenenfalls die erste Flüssigkeit, ausgeübt werden.

Gemäß einer Weiterbildung des erfindungsgemäßen Verfahrens die folgenden weiteren Schritte vorgesehen sein:
F) Unterbrechen des Auspressens der ersten Flüssigkeit und der zweiten Flüssigkeit;
G) Verschließen der zumindest zwei Nebenleitungen durch die von dem elastisch verformten zumindest einen gummielastischen Körper wirkende Rückstellkraft;
H) Aushärten des Flüssigkeitsgemischs in dem Rohr und, sofern dort enthalten, auch in der Hauptleitung unter Bildung eines Pfropfens;
I) Erneutes Auspressen der ersten Flüssigkeit und der zweiten Flüssigkeit;
J) Ausstoßen des Pfropfens durch das Rohr aufgrund des hydraulischen Drucks der ersten Flüssigkeit in der Hauptleitung;
K) Öffnen der zumindest zwei Nebenleitungen durch eine elastische Verformung des zumindest einen gummielastischen Körpers aufgrund einer von der zweiten Flüssigkeit ausgeübten und hydraulisch vermittelten Kraft auf die Wandungen der zumindest zwei Nebenleitungen;
L) Einspritzen der zweiten Flüssigkeit in die erste Flüssigkeit in der Hauptleitung durch zumindest eine Einmündung der offenen zumindest zwei Nebenleitungen in die Hauptleitung und dadurch Vermischen der ersten Flüssigkeit mit der zweiten Flüssigkeit; und
M) Auspressen des Flüssigkeitsgemischs durch das an die Hauptleitung angeschlossene Rohr.

Hierdurch kann das Verfahren unterbrochen werden, aber die Hauptleitung und die Nebenleitungen auch nach dem Aushärten noch zum Herstellen von Flüssigkeitsgemisch aus der ersten und der zweiten Flüssigkeit verwendet werden.

Es kann auch vorgesehen sein, dass die Hauptleitung in dem zumindest einen gummielastischen Körper ausgeformt ist oder in einem weiteren gummielastischen Körper ausgeformt ist wobei in Schritt C) ein Öffnen der Hauptleitung durch eine elastische Verformung des zumindest einen gummielastischen Körpers oder des weiteren gummielastischen Körpers aufgrund einer von der ersten Flüssigkeit ausgeübten und hydraulisch vermittelten Kraft auf die Wandungen der Hauptleitung erfolgt und in Schritt D) das Einspritzen der zweiten Flüssigkeit in die erste Flüssigkeit in die geöffnete Hauptleitung durch die mehreren einander gegenüberliegenden Einmündungen der offenen zumindest zwei Nebenleitungen in die geöffnete Hauptleitung erfolgt.

Hierdurch kann das Flüssigkeitsgemisch durch die sich schließende Hauptleitung in das Rohr gedrückt werden, von wo aus es gut mit einem hydraulisch vermittelten Druck der ersten Flüssigkeit auch nach Aushärten ausgestoßen werden kann.

Des Weiteren kann vorgesehen sein, dass in Schritt G) ein Verschließen der Hauptleitung durch die von dem elastisch verformten zumindest einen gummielastischen Körper wirkende Rückstellkraft oder durch die von dem elastisch verformten weiteren gummielastischen Körper wirkende Rückstellkraft erfolgt, in Schritt K) ein Öffnen der Hauptleitung durch eine elastische Verformung des zumindest einen gummielastischen Körpers oder des weiteren gummielastischen Körpers aufgrund einer von der ersten Flüssigkeit ausgeübten und hydraulisch vermittelten Kraft auf die Wandungen der Hauptleitung erfolgt und in Schritt L) das Einspritzen der zweiten Flüssigkeit in die erste Flüssigkeit in die geöffnete Hauptleitung durch zumindest eine Einmündung der offenen zumindest zwei Nebenleitungen in die geöffnete Hauptleitung erfolgt.

Hierdurch kann das Flüssigkeitsgemisch durch die sich schließende Hauptleitung in das Rohr gedrückt werden, von wo aus es gut mit einem hydraulisch vermittelten Druck der ersten Flüssigkeit auch nach Aushärten ausgestoßen werden kann.

Es ist bei dem erfindungsgemäßen Verfahren vorgesehen, dass zumindest zwei Nebenleitungen vorhanden sind und in Schritt D) das Einspritzen der zweiten Flüssigkeit in die erste Flüssigkeit in der Hauptleitung durch mehrere einander gegenüberliegende Einmündungen der offenen zumindest zwei Nebenleitungen in die Hauptleitung erfolgt, wobei die Ströme der eingespritzten zweiten Flüssigkeit in der Hauptleitung aufeinandertreffen und dadurch das Vermischen der ersten Flüssigkeit mit der zweiten Flüssigkeit gefördert wird.

Hierdurch wird eine schnellere und stärkere Durchmischung der ersten Flüssigkeit mit der zweiten Flüssigkeit zur Herstellung des Flüssigkeitsgemischs erreicht.

Der Erfindung liegt die überraschende Erkenntnis zugrunde, dass es mit zumindest einer elastisch verschließbaren Nebenleitung zum Zuführen der zweiten Flüssigkeit in eine Hauptleitung, in der die erste Flüssigkeit geleitet wird, gelingt, die zwei Flüssigkeiten ausreichend gut zu vermischen, wobei ein Pfropfen des ausgehärteten Flüssigkeitsgemischs durch einen Druck auf die erste Flüssigkeit aus der Vorrichtung ausgestoßen werden kann, weil die zumindest eine Nebenleitung an der zumindest einen Einmündung in die Hauptleitung elastisch verschließbar ist, so dass sich in dem Mischbereich keine Hinterschneidungen des Pfropfens ausbilden, die einen Ausstoß des Pfropfens verhindern würden. Die zumindest eine Nebenleitung kann dabei durch einen Druck auf die zweite Flüssigkeit hydraulisch geöffnet werden. Die Erfindung basiert auch auf der überraschenden Erkenntnis, dass die Flüssigkeiten sich auch ohne statischen Mischer mit Hinterschneidungen ausreichend miteinander vermischen lassen, wenn die zweite Flüssigkeit seitlich in die erste Flüssigkeit in der Hauptleitung eingespritzt wird. Dadurch, dass die zumindest eine Nebenleitung erst öffnet, wenn ein ausreichender hydraulischer Druck mit der zweiten Flüssigkeit aufgebaut wurde, ist sichergestellt, dass die zweite Flüssigkeit mit einem kräftigen Stoß in die erste Flüssigkeit eingespritzt wird. Der stoßartige und nur mit großer Geschwindigkeit mögliche Eintrag der zweiten Flüssigkeit in die erste Flüssigkeit, erzeugt starke Verwirbelungen, die sicherstellen, dass sich die erste Flüssigkeit und die zweite Flüssigkeit in der Hauptleitung ausreichend stark mischen, auch ohne dass hierfür komplexe Strukturen von statischen Mischern vorhanden sind. Hierfür dürfen die erste und die zweite Flüssigkeit keine zu hohe Viskosität haben. Mit dem gummielastischen Körper, der zumindest die zumindest eine Nebenleitung begrenzt, und vorzugsweise auch die Hauptleitung begrenzt, kann sichergestellt werden, dass die zweite Flüssigkeit mit einem hohen Impuls aufgrund des notwendigen Mindestdrucks in die erste Flüssigkeit eingespritzt wird.

Die erfindungsgemäße Vorrichtung ist dabei so aufgebaut, dass keine Mischwendeln zur Vermischung der Flüssigkeiten notwendig sind. Dadurch ist es möglich, einen teilweise oder vollständig in der Mischvorrichtung ausgehärteten Klebstoff auszustoßen und anschließend die flüssigen Klebstoffkomponenten weiterhin zu vermischen und auszutragen.

Die erfindungsgemäße Vorrichtung und das erfindungsgemäße Verfahren funktionieren in der Weise, dass die erste Flüssigkeit in die Hauptleitung gefördert wird. Gleichzeitig wird die zweite Flüssigkeit aus der zumindest einen Zuleitung gegen die zumindest eine Nebenöffnung des zumindest einen gummielastischen Körpers gepresst, bis sich ein solcher Druck aufgebaut hat, dass sich die zumindest eine Nebenleitung bis zu der zumindest einen Einmündung öffnet und die zweite Flüssigkeit durch die zumindest einen Einmündung in die Hauptleitung gespritzt wird. Dort treffen die Flüssigkeitsstrahlen der zweiten Flüssigkeit aufeinander und/oder auf eine Wandung der Hauptleitung und verteilen sich auf Grund des Aufpralls über den Querschnitt der Hauptleitung in der ersten Flüssigkeit. Dadurch erfolgt die Vermischung der ersten Flüssigkeit und der zweiten Flüssigkeit miteinander.

Vorteilhaft ist es dabei, wenn der Querschnitt der Hauptleitung möglichst klein ist. Wenn das Nachpressen der ersten Flüssigkeit und der zweiten Flüssigkeit eingestellt wird, dann kann das Flüssigkeitsgemisch in der Hauptleitung, im Bereich der zumindest einen Einmündung und im Innenraum des Rohrs aushärten. Nach Beendigung der Druckbeaufschlagung der zumindest einen Nebenleitung durch die zweite Flüssigkeit schließt sich die zumindest eine Nebenleitung. Bei erneutem Auspressen der ersten und der zweiten Flüssigkeiten wird durch die Druckbeaufschlagung der zweiten Flüssigkeit die zumindest eine Nebenleitung wieder geöffnet und das miteinander vermischte und ausgehärtete Flüssigkeitsgemisch wird als Pfropfen beziehungsweise in Form von ausgehärtetem Klebstoff durch die nachströmende erste Flüssigkeit aus der Hauptleitung und aus dem Innenraum des Rohrs ausgepresst. Danach und dabei wird die nachströmende zweite Flüssigkeit wieder mit der eingespritzten ersten Flüssigkeit vermischt.

Eine beispielhafte erfindungsgemäße Vorrichtung kann zusammengesetzt sein aus
a) einem Rohr mit einem Innendurchmesser kleiner 1,5 mm,
b) einer Hauptzuleitung, die zu dem Rohr führt und die mit einem Hauptreservoir verbunden ist oder verbindbar ist, das eine erste Flüssigkeit enthält,
c) einer gummielastischen Hülse als den gummielastischen Körper, die einen axialen Hohlraum als Hauptleitung enthält, wobei die gummielastische Hülse mindestens zwei Einspritzöffnungen als Einmündungen in den Hohlraum besitzt, die als Nebenleitungen von der äußeren Mantelfläche der Hülse zum axialen Hohlraum der Hülse ausgeführt sind, wobei die mindestens zwei Einspritzöffnungen im nicht mit Druck beaufschlagten Zustand verschlossen sind,
d) wobei ein proximales Ende des Rohrs in die gummielastische Hülse so eingesteckt ist, dass das proximale Ende des Rohrs nicht die Einspritzöffnungen überdeckt,
e) einem Mantelrohr, das als Mantel die gummielastische Hülse koaxial umschließt, so dass eine flüssigkeitsdurchlässiger Zwischenraum zwischen der äußeren Mantelfläche der Hülse und der inneren Mantelfläche des Mantelrohrs gebildet ist, wobei der Zwischenraum mit einem Nebenreservoir verbunden ist oder verbindbar ist, das die zweite Flüssigkeit enthält, und
f) wobei die mindestens zwei Einspritzöffnungen der gummielastischen Hülse durch Druckbeaufschlagung mit der zweiten Flüssigkeit zu öffnen sind.

Die Vorrichtung funktioniert in der Weise, dass die erste Flüssigkeit in die Hauptleitung beziehungsweise in den axialen Hohlraum der Hülse gefördert wird. Gleichzeitig wird die zweite Flüssigkeit aus der zumindest einen Zuleitung gegen die zumindest eine Nebenöffnung des zumindest einen gummielastischen Körpers beziehungsweise in dem Zwischenraum gegen die Hülse gepresst, bis sich ein solcher Druck aufgebaut hat, dass sich die zumindest eine Nebenleitung bis zu der zumindest einen Einmündung beziehungsweise dass sich die Einspritzöffnungen öffnen und die zweite Flüssigkeit durch die zumindest einen Einmündung beziehungsweise durch die Einspritzöffnungen in die Hauptleitung beziehungsweise den axialen Hohlraum gespritzt wird. Dort treffen die Flüssigkeitsstrahlen der zweiten Flüssigkeit aufeinander oder auf eine Wandung der Hauptleitung und verteilen sich auf Grund des Aufpralls über den Querschnitt der Hauptleitung beziehungsweise des axialen Hohlraums der Hülse. Dadurch erfolgt die Vermischung der ersten Flüssigkeit und der zweiten Flüssigkeit miteinander.

Vorteilhaft ist es dabei, wenn der Querschnitt der Hauptleitung beziehungsweise des axialen Hohlraums möglichst klein ist. Wenn das Nachpressen der ersten Flüssigkeit und der zweiten Flüssigkeit eingestellt wird, dann können die vermischten Flüssigkeiten (das Flüssigkeitsgemisch) in der Hauptleitung beziehungsweise im axialen Hohlraum, im Bereich der zumindest einen Einmündung beziehungsweise der Einspritzdüsen und im Innenraum des Rohrs aushärten. Nach Beendigung der Druckbeaufschlagung der zumindest einen Nebenleitung beziehungsweise der Einspritzöffnungen der Hülse durch die zweite Flüssigkeit schließt sich die zumindest eine Nebenleitung beziehungsweise schließen sich die Einspritzöffnungen. Bei erneutem Auspressen der ersten und der zweiten Flüssigkeiten werden durch die Druckbeaufschlagung der zweiten Flüssigkeit die zumindest eine Nebenleitung beziehungsweise die Einspritzöffnungen wieder geöffnet und die miteinander vermischten und ausgehärteten Flüssigkeiten werden als Pfropfen oder in Form von ausgehärtetem Klebstoff durch die nachströmende erste Flüssigkeit aus der Hauptleitung beziehungsweise dem Hohlraum der Hülse und aus dem Innenraum des Rohrs ausgepresst. Danach und dabei wird die nachströmende zweite Flüssigkeit wieder mit der eingespritzten ersten Flüssigkeit vermischt.

Ein beispielhaftes erfindungsgemäßes Verfahren zur Herstellung eines Flüssigkeitsgemischs kann die folgenden nacheinander ablaufenden Schritte umfassen:
a) Öffnen einer Zweikomponenten-Kartusche, die ein Hauptreservoir aufweist, das mit einer ersten Flüssigkeit gefüllt ist, und die ein separates Nebenreservoir aufweist, das mit einer zweiten Flüssigkeit gefüllt ist,
b) Verbinden der oben genannten beispielhaften Vorrichtung mit der geöffneten Zweikomponenten-Kartusche,
c) Herauspressen der beiden Flüssigkeiten aus dem Hauptreservoir und dem Nebenreservoir durch axiale Bewegung von Kolben in Richtung eines Kartuschenkopfs der Zweikomponenten-Kartusche,
d) Druckbeaufschlagung der gummielastischen Hülse durch die zweite Flüssigkeit,
e) Öffnen der mindestens zwei Einspritzöffnungen der gummielastischen Hülse durch Druckbeaufschlagung der zweiten Flüssigkeit,
f) Fließen der ersten Flüssigkeit in den axialen Hohlraum der Hülse,
g) Einspritzen der zweiten Flüssigkeit aus den Einspritzöffnungen der Hülse in die erste Flüssigkeit,
h) Vermischen der zweiten Flüssigkeit mit der ersten Flüssigkeit im axialen Hohlraum der Hülse und dem Innenraum des Rohrs, und
i) Auspressen des Flüssigkeitsgemischs aus dem Rohr durch die nachströmende erste und zweite Flüssigkeit.

Bei dem beispielhaften Verfahren kann vorgesehen sein, dass nach dem Schritt i) das Auspressen der ersten und der zweiten Flüssigkeit beendet wird, und nachfolgend die die folgenden Schritte durchgeführt werden:
j) Aushärten der vermischten Flüssigkeiten im Rohr und dem axialen Hohlraum in Flussrichtung nach den Einspritzdüsen und auf Höhe der Einspritzdüsen,
k) erneutes Auspressen der Flüssigkeiten aus den Kartuschen,
l) Auspressen des ausgehärteten Flüssigkeitsgemischs mit der zweiten Flüssigkeit aus den Einspritzöffnungen,
m) Herauspressen des ausgehärteten Flüssigkeitsgemischs mit der ersten Flüssigkeit durch das Rohr, und
n) Wiederholung der Schritte c) bis i) und optional auch der Schritte j) bis n).

Im Folgenden werden weitere Ausführungsbeispiele der Erfindung anhand von zweiundzwanzig schematisch dargestellten Figuren erläutert, ohne jedoch dabei die Erfindung zu beschränken. Dabei zeigt:
Figur 1: eine schematische Querschnittansicht einer Ausschnittvergrößerung einer beispielhaften erfindungsgemäßen Vorrichtung zum Mischen von Flüssigkeiten während eines Mischvorgangs;
Figur 2: eine schematische Querschnittansicht einer Ausschnittvergrößerung der Vorrichtung nach Figur 1 mit geschlossenen Nebenleitungen;
Figur 3: eine schematische Querschnittansicht einer Ausschnittvergrößerung der Vorrichtung nach den Figuren 1 mit geschlossenen Haupt- und Nebenleitungen;
Figur 4: eine schematische Querschnittansicht einer Ausschnittvergrößerung der Vorrichtung nach den Figuren 1 mit geöffneten Haupt- und Nebenleitungen;
Figur 5: eine schematische Querschnittansicht der gesamten Vorrichtung vor dem Mischen;
Figur 6: eine schematische Querschnittansicht der gesamten Vorrichtung nach dem Mischen;
Figur 7: eine schematische Querschnittansicht der gesamten Vorrichtung während der Lagerung;
Figur 8: eine schematische perspektivische Querschnittansicht eines Ausschnitts der Vorrichtung mit geöffneten Leitungen;
Figur 9: eine schematische perspektivische Querschnittansicht eines Ausschnitts der Vorrichtung mit geschlossenen Leitungen;
Figur 10: eine schematische perspektivische Teilquerschnittansicht eines Ausschnitts der Vorrichtung während der Lagerung;
Figur 11: eine schematische perspektivische Teilquerschnittansicht der Vorrichtung während der Lagerung;
Figur 12: eine schematische perspektivische Querschnittansicht der Vorrichtung nach dem Mischvorgang;
Figur 13: eine schematische perspektivische Außenansicht der Vorrichtung mit getrenntem Verschluss;
Figur 14: eine schematische perspektivische Außenansicht der Vorrichtung mit angeschlossenem Mischer;
Figur 15: eine schematische perspektivische Außenansicht der Vorrichtung mit befestigtem Mischer;
Figur 16: eine schematische perspektivische Außenansicht einer alternativen erfindungsgemäßen Vorrichtung während der Lagerung;
Figur 17: eine schematische perspektivische Außenansicht der alternativen Vorrichtung nach Figur 16 bereit zum Mischen;
Figur 18: eine schematische perspektivische Teilquerschnittansicht eines Teils der alternativen Vorrichtung nach den Figuren 16 und 17;
Figur 19: eine schematische perspektivische Querschnittansicht der alternativen Vorrichtung nach Figur 16;
Figur 20: eine schematische perspektivische Querschnittansicht der alternativen Vorrichtung nach den Figuren 16 bis 19 während dem Mischen;
Figur 21: eine schematische Querschnittansicht der alternativen Vorrichtung nach den Figuren 16 bis 20 während dem Mischen;
Figur 22: eine Ausschnittvergrößerung als schematische Querschnittansicht der alternativen Vorrichtung nach den Figuren 16 bis 21 am Ende des Mischvorgangs;
Figur 23: eine schematische Querschnittansicht einer weiteren alternativen erfindungsgemäßen Vorrichtung mit geschlossenen Nebenleitungen in mehreren gummielastischen Körpern; und
Figur 24: eine schematische Querschnittansicht der weiteren alternativen Vorrichtung nach Figur 23 mit geöffneten Nebenleitungen.

In den Figuren werden auch für unterschiedliche Ausführungsbeispiele die gleichen Bezugszeichen verwendet, da sich die Ausführungsbeispiele nur geringfügig unterscheiden und gleichzeitig die Vergleichbarkeit der Ausführungsbeispiele verbessert wird.

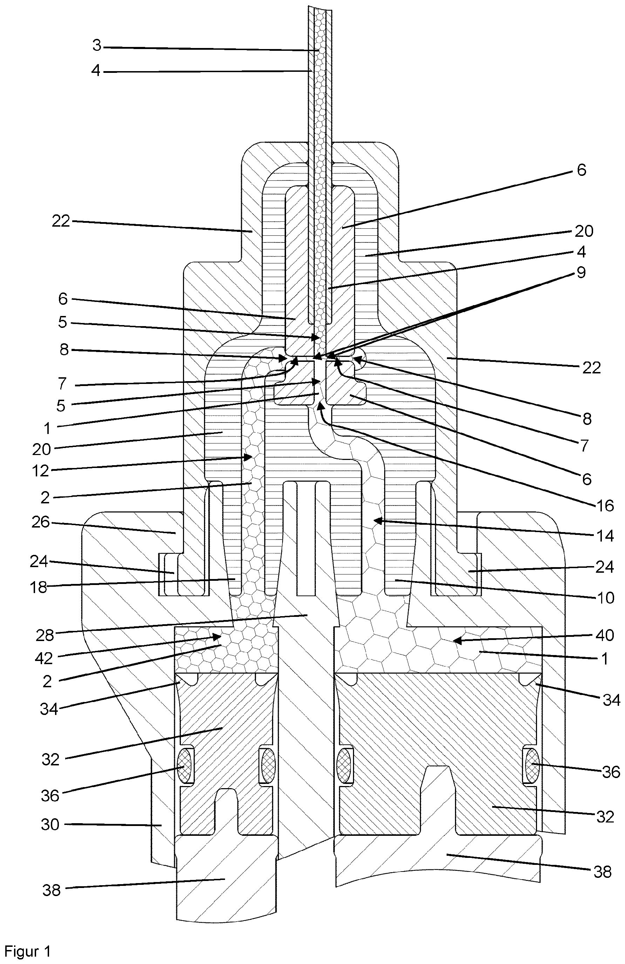

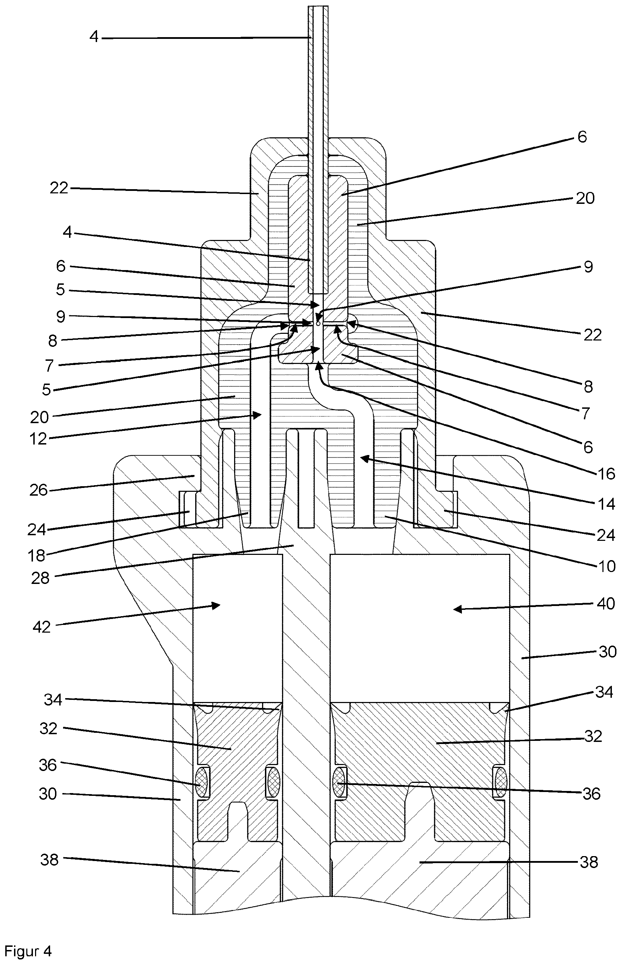

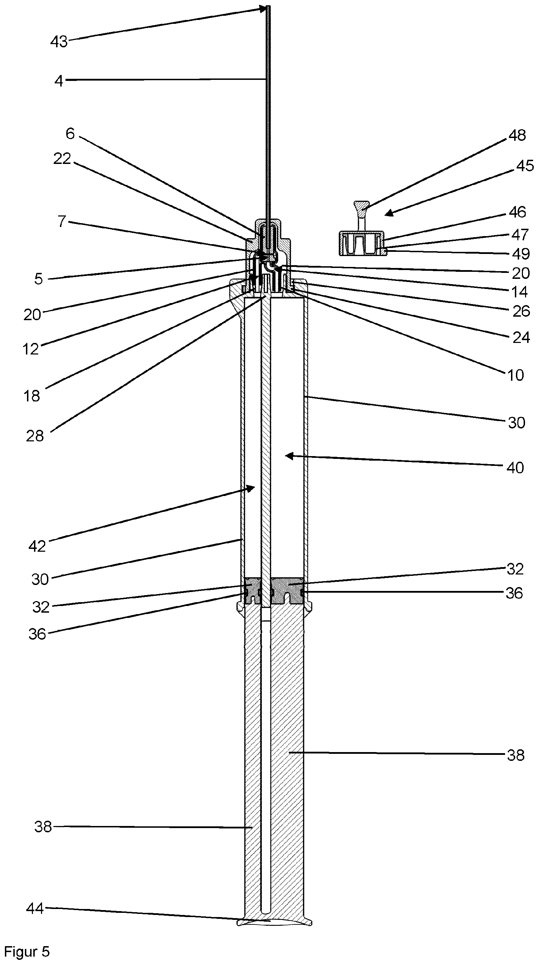

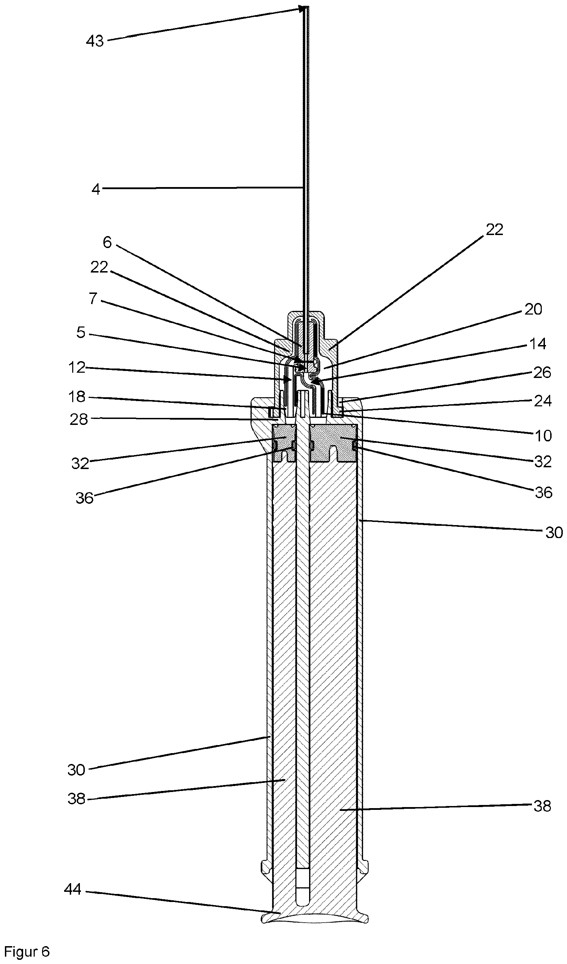

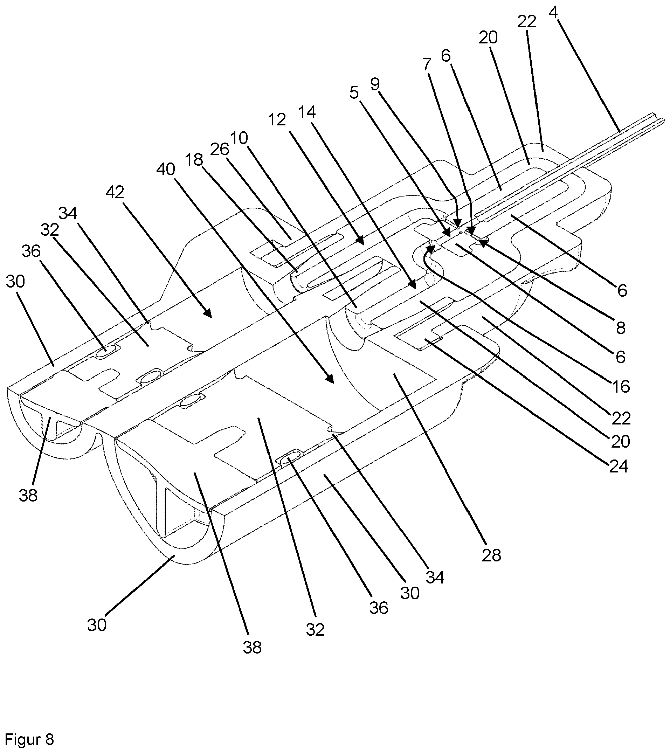

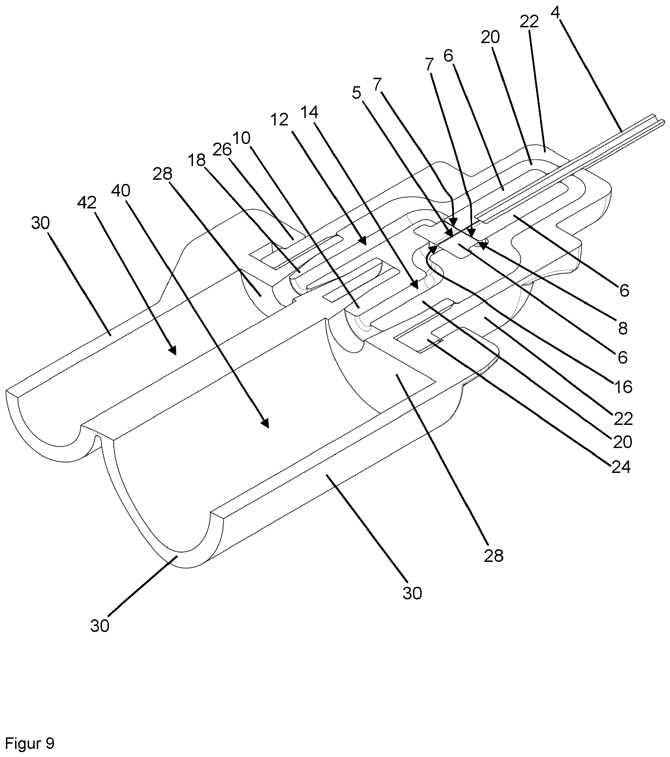

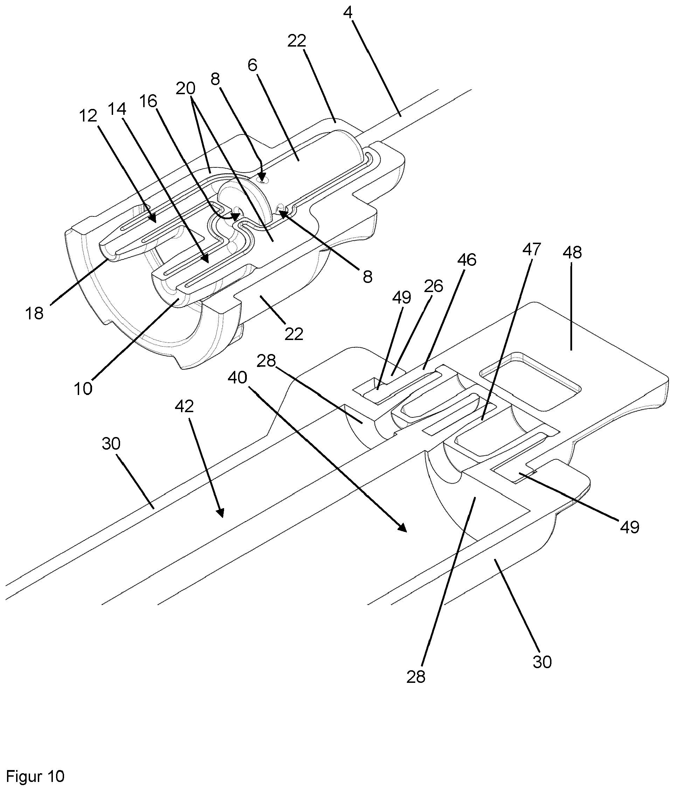

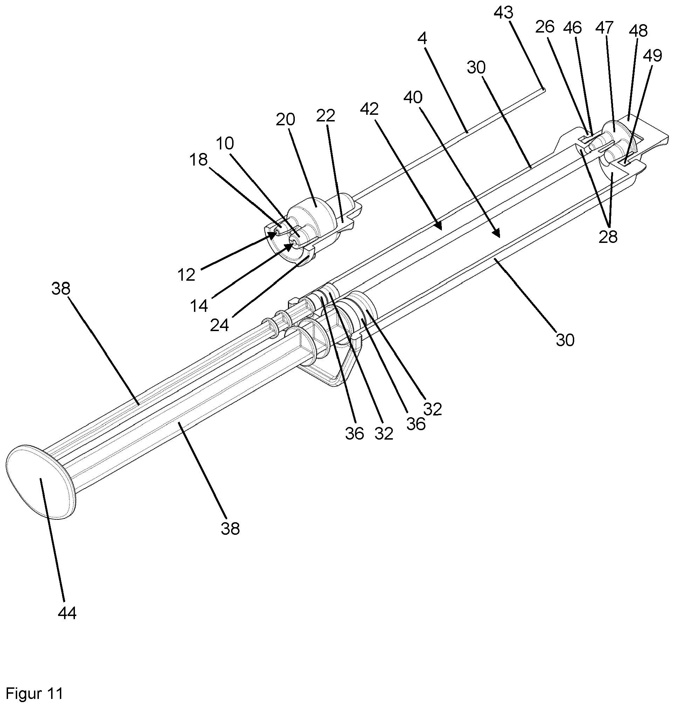

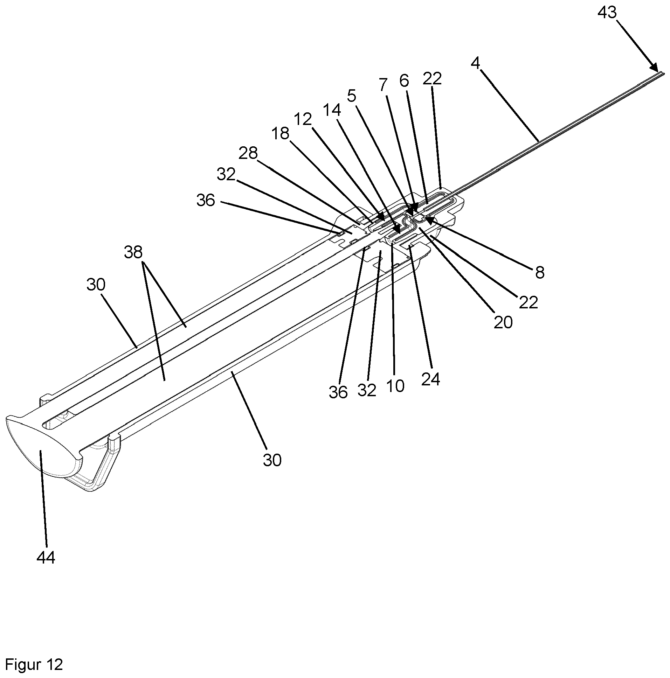

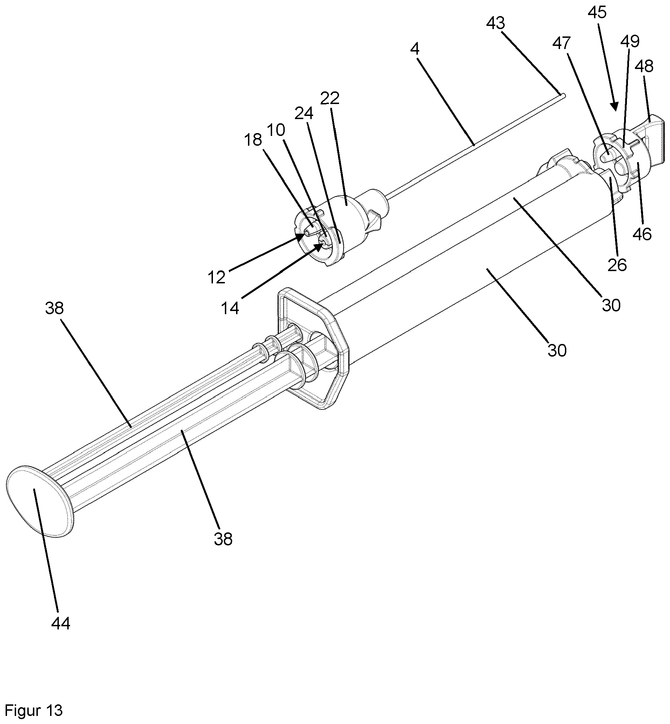

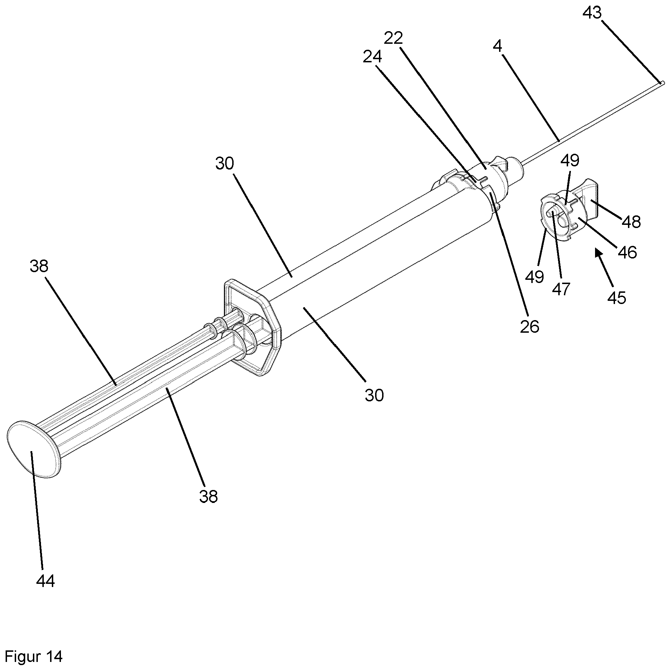

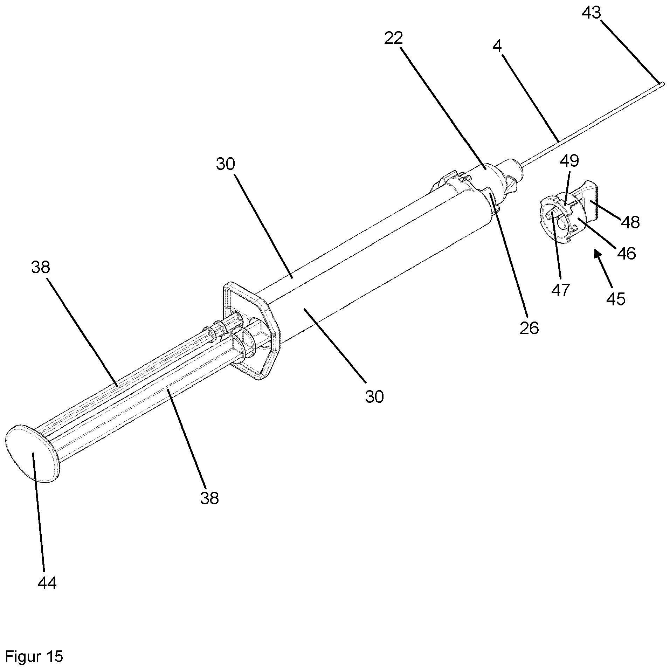

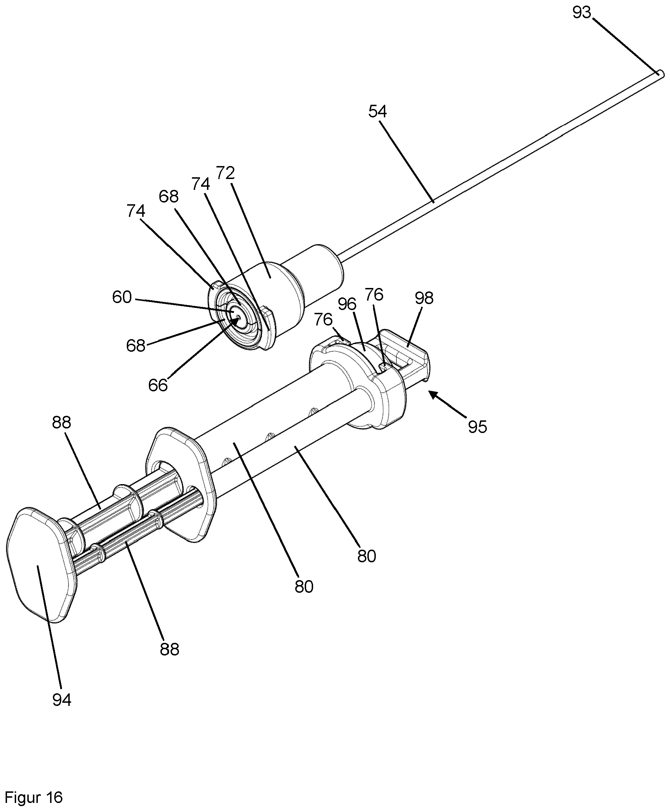

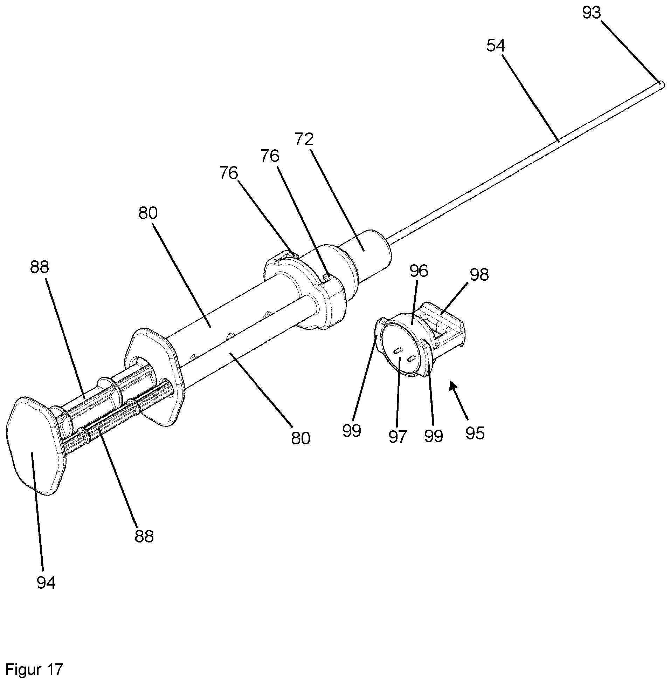

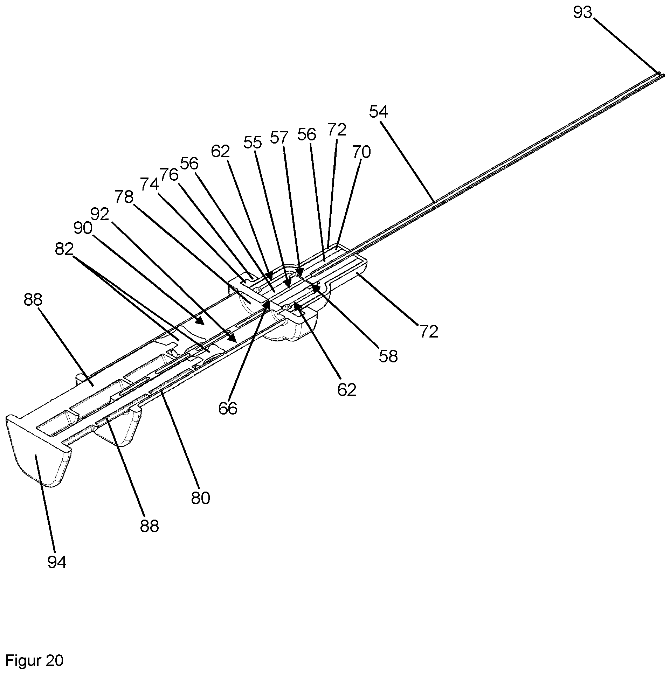

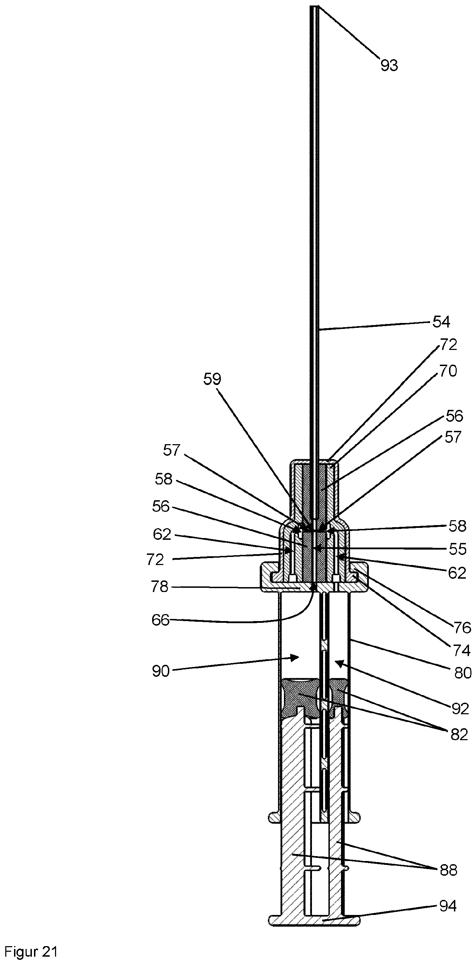

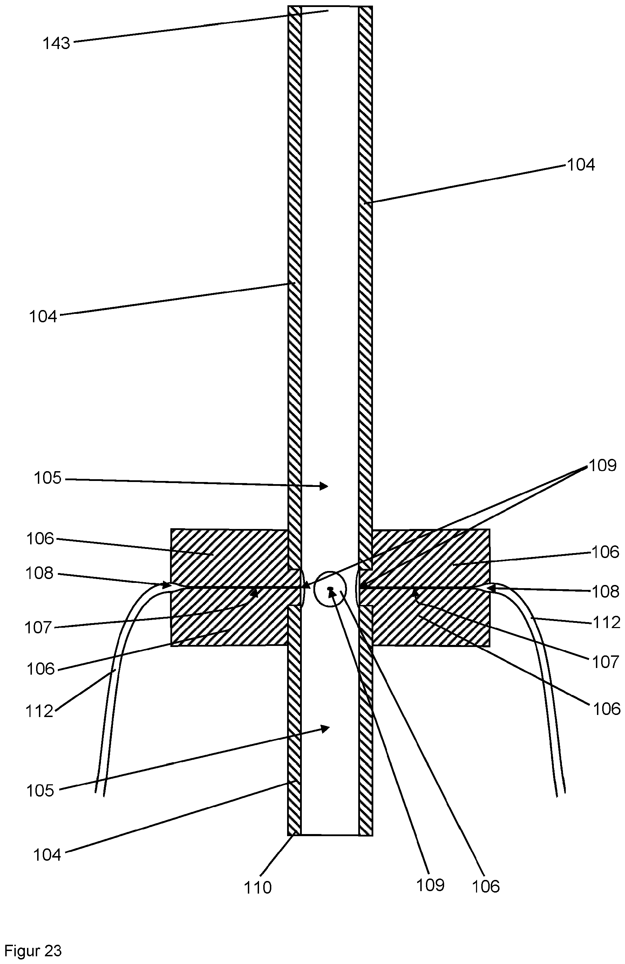

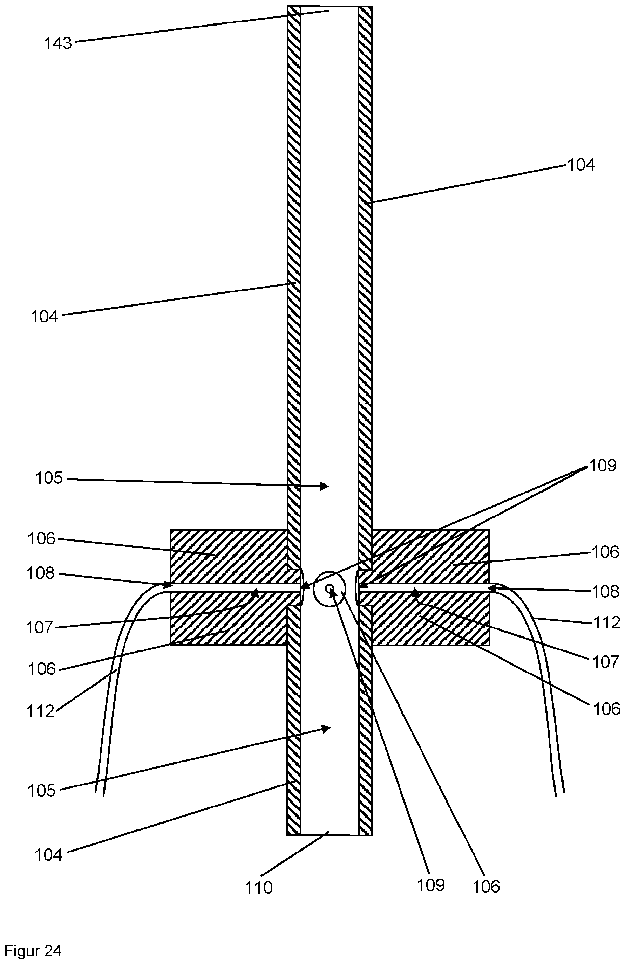

In den Figuren 1 bis 15 sind Abbildungen eines ersten Ausführungsbeispiels einer erfindungsgemäßen Vorrichtung zum Mischen der Flüssigkeiten und Teile davon in verschiedenen Ansichten gezeigt. Die Figuren 16 bis 22 zeigen ein alternatives zweites Ausführungsbeispiel einer erfindungsgemäßen Vorrichtung. Die Figuren 23 und 24 zeigen schematische Querschnittansichten eines weiteren alternativen dritten Ausführungsbeispiels.

Die erste erfindungsgemäße Vorrichtung nach den Figuren 1 bis 15 ist dazu vorgesehen, eine erste Flüssigkeit 1 als Hauptkomponente (in Figur 1 durch eine Wabenstruktur mit großen Waben dargestellt) und eine zweite Flüssigkeit 2 als Nebenkomponente (in Figur 1 durch eine Wabenstruktur mit mittelgroßen Waben dargestellt) miteinander zu mischen. Dabei entsteht ein Flüssigkeitsgemisch 3 (in Figur 1 durch eine Wabenstruktur mit kleinen Waben dargestellt), das durch ein Rohr 4 ausgetragen werden kann, so wie das in Figur 1 gezeigt ist. Das Rohr 4 besteht vorzugsweise aus einem Kunststoff, der sich nicht mit dem Flüssigkeitsgemisch 3 verbindet, auch dann nicht, wenn das Flüssigkeitsgemisch 3 in dem Rohr 4 aushärtet.

Das Rohr 4 kann selbst gerade sein und kann in gerader Linie mit einer Hauptleitung 5 verbunden sein, der in einem gummielastischen Körper 6 ausgeformt ist. Die Hauptleitung 5 selbst ist vorzugsweise ebenfalls gerade, um einen Ausstoß von ausgehärtetem Flüssigkeitsgemisch 3 aus der Hauptleitung 5 und dem Rohr 4 zu erleichtern. Der gummielastische Körper 6 kann aus einem Elastomer bestehen oder aufgebaut werden. Geeignete Elastomere sind beispielsweise EPDM, Silikonkautschuk, Naturkautschuk, Butadien-Acrylnitril-Kautschuk und synthetischer Polyisoprenkautschuk. Der gummielastische Körper 6 kann als Hülse ausgeführt sein, wobei vorzugsweise in dem Inneren der Hülse die Hauptleitung 5 als axialer Kanal geführt sein kann.

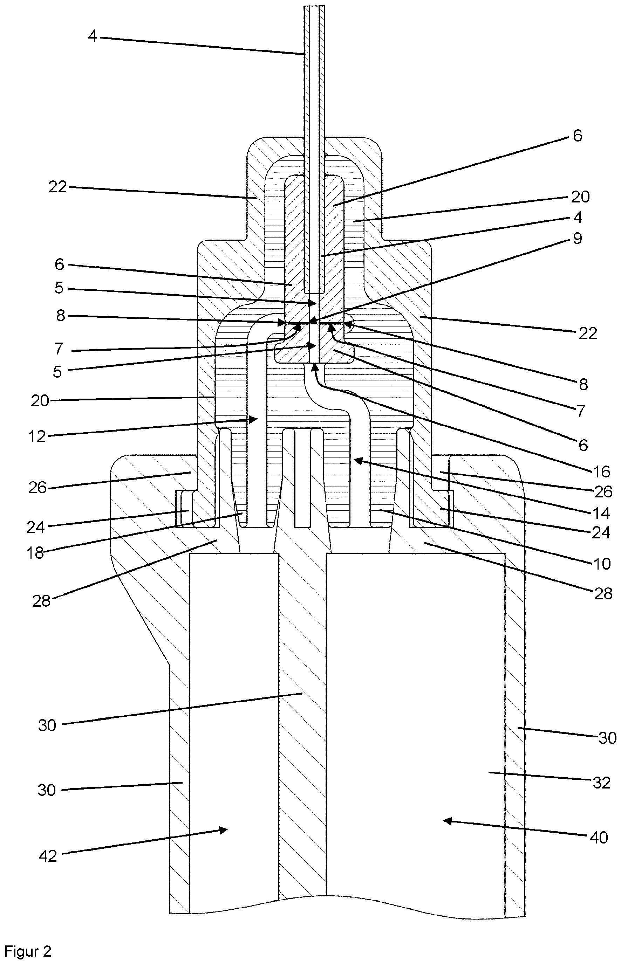

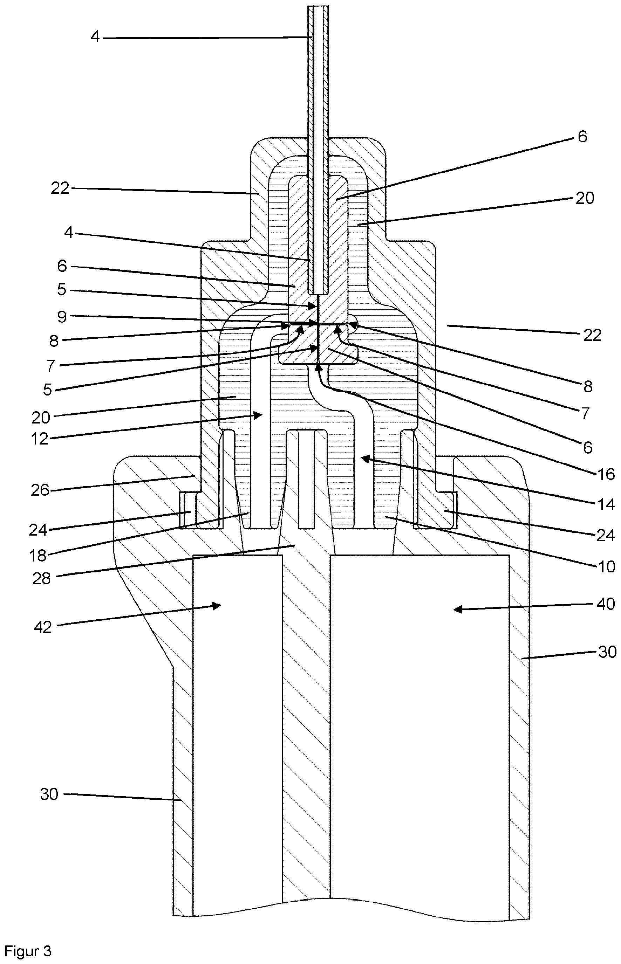

In dem gummielastischen Körper 6 können zusätzlich vier Nebenleitungen 7 ausgeformt sein, die sich in gerader Linie radial von der Hauptleitung 5 weg bis zu einer Oberfläche des gummielastischen Körpers 6 erstrecken. Hierzu können in einer Mantelfläche des hülsenförmigen gummielastischen Körpers 6 vier Nebenöffnungen 8 angeordnet sein, die in die Nebenleitungen 7 und damit in den gummielastischen Körper 6 hineinführen. Die Nebenöffnungen 8 können konisch geformt oder auf andere Weise geweitet sein, um ein Einpressen der zweiten Flüssigkeit 2 in die Nebenleitungen 7 zu erleichtern, wenn die Nebenleitungen 7 geschlossen sind (siehe Figuren 2 und 3).

Die Nebenleitungen 7 münden über Einmündungen 9 in die Hauptleitung 5. Die Nebenleitungen 7 können durch ein Durchstechen des gummielastischen Körpers 6 ausgeformt werden. Wesentlich ist, dass die Nebenleitungen 7 im entspannten Zustand des gummielastischen Körpers 6, also ohne dass eine Kraft beziehungsweise ein Druck auf die Leitungswandungen der Nebenleitungen 7 wirkt, verschlossen sind (siehe Figuren 2 und 3). In gleicher Weise kann vorgesehen sein, dass auch die Hauptleitung 5 im entspannten, also druckfreien Zustand verschlossen ist (siehe Figur 3).

Die Einmündungen 9 können alle an einem Ort in die Hauptleitung 5 münden. Hierdurch treffen Flüssigkeitsstrahlen der zweiten Flüssigkeit 2, die durch die Einmündungen 9 in die Hauptleitung 5 eingespritzt werden, aufeinander und die kinetische Energie der Flüssigkeitsstrahlen kann so zur Durchmischung der zweiten Flüssigkeit 2 mit der ersten Flüssigkeit 1 genutzt werden.

Das Rohr 4 kann in die Hauptleitung 5 eingesteckt sein, wobei das Rohr 4 nur so tief in die Hauptleitung 5 eingesteckt ist, dass es keine der Einmündungen 9 überdeckt. Theoretisch könnten auch Durchbrechungen in der Wandung des Rohrs 4 angeordnet sein, durch die die Nebenleitungen 7 über die Einmündungen 9 in die Hauptleitung münden.

Die erste Flüssigkeit 1 kann über einen Hauptanschluss 10 in die Hauptleitung 5 eingepresst werden. Der Hauptanschluss 10 kann hierzu flüssigkeitsleitend und druckdicht mit der Hauptleitung 5 verbunden sein. Zum Zuführen der zweiten Flüssigkeit 2 zu den Nebenleitungen 7 kann eine Zuleitung 12 vorgesehen sein. Die Zuleitung 12 kann über einen die Nebenöffnungen 8 umschließenden ringförmigen Kanal mit den Nebenleitungen 7 flüssigkeitsleitend und druckdicht verbunden sein, um sicherzustellen, dass die zweite Flüssigkeit 2 durch alle Nebenleitungen 7 in die Hauptleitung 5 eingespritzt werden kann.

Analog kann eine Hauptleitung 14 vorgesehen sein, die die Hauptleitung 5 ausgehend von einer Hauptöffnung 16 mit dem Hauptanschluss 10 verbindet. Die Hauptöffnung 16 kann konisch geformt oder auf andere Weise geweitet sein, um ein Einpressen der ersten Flüssigkeit 1 in die Hauptleitung 5 zu erleichtern, wenn sie geschlossen ist (siehe Figur 3).

Die Zuleitung 12 kann an dem den Einmündungen 8 gegenüberliegenden Ende mit einem Nebenanschluss 18 verbunden sein, über den die zweite Flüssigkeit 2 in die Zuleitung 12 eingepresst werden kann. Der Hauptanschluss 10 und der Nebenanschluss 18 können als vorstehende rohrförmige Stutzen mit konisch zulaufendem Ende geformt sein. Die Stutzen können in passende Öffnungen einer Zweikomponenten-Kartusche eingesteckt sein oder werden.

Der Hauptanschluss 10 und der Nebenanschluss 18 können einteilig mit einem Innenteil 20 ausgeführt sein. Das Innenteil 20 kann aus einem Kunststoff bestehen. Die Zuleitung 12 und die Hauptzuleitung 14 können im Inneren des Innenteils 20 ausgeformt sein. Zudem kann das Innenteil 20 eine Ausnehmung zur Aufnahme des gummielastischen Körpers 6 aufweisen, in der der gummielastische Körper 6 eingebaut ist. Das Innenteil 20 kann bis auf die den Hauptanschluss 10 und den Nebenanschluss 18 bildenden Stutzen außen rotationssymmetrisch geformt sein. Hierdurch kann das Innenteil 20 gegen ein Gehäuse 22, das das Innenteil 20 umschließt, gedreht werden. Das Gehäuse 22 kann an seiner zur Zweikomponenten-Kartusche weisenden Seite Vorsprünge 24 aufweisen, die mit einer übergreifenden Aufnahme 26 an der Zweikomponenten-Kartusche nach Art eines Bajonett-Verschlusses verbunden werden können. Die Zweikomponenten-Kartusche kann Teil der erfindungsgemäßen Vorrichtung sein.

Die Zweikomponenten-Kartusche kann an ihrem vorderen Ende, das zum Verbinden mit dem Gehäuse 22, dem Hauptanschluss 10 und dem Nebenanschluss 18 vorgesehen ist, einen Kartuschenkopf 28 aufweisen. In dem Kartuschenkopf 28 können zwei Öffnungen angeordnet sein, die mit dem Hauptanschluss 10 und dem Nebenanschluss 18 zu verbinden sind. Die Zweikomponenten-Kartusche kann zwei parallel zueinander angeordnete Kartuschen 30 aufweisen. Die Zweikomponenten-Kartusche kann bis auf die beiden enthaltenen Flüssigkeiten 1, 2 aus Kunststoff bestehen. In den beiden Kartuschen 30 können axial verschiebbare Austragskolben 32 angeordnet sein, mit denen die erste Flüssigkeit 1 und die zweite Flüssigkeit 2 aus der Zweikomponenten-Kartusche heraus- und in die Hauptzuleitung 14 und in die Zuleitung 12 hineingepresst werden können. Die Austragskolben 32 können eine umlaufende Abstreiflippe 34 und eine umlaufende Dichtung 36 aufweisen, um die erste Flüssigkeit 1 und die zweite Flüssigkeit 2 vollständig aus den Kartuschen 30 austreiben zu können. Die Kartusche 30 für die erste Flüssigkeit 1 kann einen größeren Innendurchmesser aufweisen als die Kartusche 30 für die zweite Flüssigkeit 2. Auf diese Weise kann ein Mischungsverhältnis erzeugt werden, bei dem ein geringerer Anteil der zweiten Flüssigkeit 2 einem größeren Volumen der ersten Flüssigkeit 1 beigemengt werden kann.

Die Austragskolben 32 können über Stößel 38 von der Rückseite der Zweikomponenten-Kartusche aus angetrieben werden. Die Kartuschen 30 weisen in ihrem Inneren zylindrische Innenräume auf, die ein Hauptreservoir 40 für die erste Flüssigkeit 1 und ein Nebenreservoir 42 für die zweite Flüssigkeit 2 begrenzen. Die erste Flüssigkeit 1 ist in dem Hauptreservoir 40 enthalten und die zweite Flüssigkeit 2 ist in dem Nebenreservoir 42 enthalten. Das Hauptreservoir 40 und das Nebenreservoir 42 sind an ihrer Vorderseite durch den Kartuschenkopf 28 und auf der Rückseite durch die Austragskolben 32 begrenzt, wobei der Inhalt mit den Austragskolben 32 durch die Öffnungen im Kartuschenkopf 28 in die Hauptzuleitung 14 und in die Zuleitung 12 gepresst werden kann.

Das fertige Flüssigkeitsgemisch 3 kann durch ein offenes Ende 43 des Rohrs 4 ausgetragen werden. Das Rohr 4 ist an dem, dem offenen Ende 43 gegenüberliegenden Ende mit der Hauptleitung 5 verbunden, beziehungsweise in die Hauptleitung 5 eingesteckt.

Die beiden Stößel 38 können an dem den Kartuschen 30 gegenüberliegenden Ende über ein Verbindungselement 44 miteinander verbunden sein. Hierdurch wird sichergestellt, dass die beiden Austragskolben 32 nur synchron vorgetrieben werden können, so dass immer das gleiche Mischungsverhältnis der ersten Flüssigkeit 1 und der zweiten Flüssigkeit 2 zum Mischen des Flüssigkeitsgemischs 3 erzeugt wird. Das Verbindungselement 44 kann auch als gemeinsames Griffstück zum manuellen Auspressen der ersten Flüssigkeit 1 und der zweiten Flüssigkeit 2 aus der Zweikomponenten-Kartusche nach Art einer Spritze genutzt werden und hierfür passend geformt sein. Hierfür kann auch an der Rückseite der Kartuschen 30 eine passende Gegenhalterung angeordnet sein.

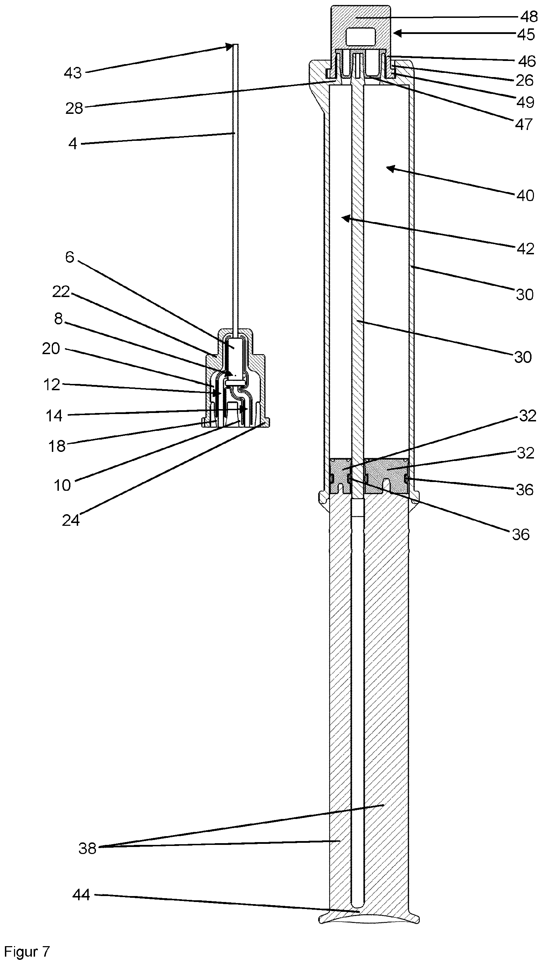

Die Zweikomponenten-Kartusche kann zur Lagerung mit einem Verschluss 45 verschlossen sein, der die beiden Öffnungen im Kartuschenkopf 28 verschließt und so die erste Flüssigkeit 1 und die zweite Flüssigkeit 2 in der Zweikomponenten-Kartusche einschließt (siehe Figuren 7, 10 und 11). Der Verschluss 45 kann eine äußere Kappe 46, einen gegen die äußere Kappe 46 verdrehbar gelagerten Innenteil 47 und einen Griff 48 aufweisen. An dem Innenteil 47 können zwei Zapfen zum Verschließen der Öffnungen im Kartuschenkopf 28 der Zweikomponenten-Kartusche angeordnet sein. Die äußere Kappe 46 kann zwei Vorsprünge 49 aufweisen, so dass der Verschluss 45 mit der äußeren Kappe 46 analog dem Gehäuse 22 mit den Aufnahmen 26 an der Zweikomponenten-Kartusche nach Art eines Bajonett-Verschlusses verbunden werden kann.

Durch den Nebenanschluss 18 kann die zweite Flüssigkeit 2 durch die Zuleitung 12 in die Nebenöffnungen 8 gepresst werden, indem die zweite Flüssigkeit 2 mit dem Austragskolben 32 aus dem Nebenreservoir 42 in die Zuleitung 12 gepresst wird. Durch den Druck auf die zweite Flüssigkeit 2 weiten sich aufgrund einer elastischen Verformung des gummielastischen Körpers 6 die Nebenleitungen 7 und die Nebenleitungen 7 werden durchlässig für die zweite Flüssigkeit 2. Schließlich öffnen sich auch die Einmündungen 9 in die Hauptleitung 5 und die zweite Flüssigkeit 2 wird unter Druck in die in der Hauptleitung 5 geführte erste Flüssigkeit 1 eingespritzt. Die kinetische Energie, mit der die zweite Flüssigkeit 2 eingespritzt wird, und dadurch, dass die Flüssigkeitsströme der zweiten Flüssigkeit 2 aufeinandertreffen, wird eine starke Durchmischung der ersten Flüssigkeit 1 und der zweiten Flüssigkeit 2 zu dem Flüssigkeitsgemisch 3 bewirkt.

In gleicher Weise, wie die Nebenleitungen 7 durch einen Druck auf die zweite Flüssigkeit 2 geöffnet werden können, kann auch die Hauptleitung 5 durch einen Druck auf die erste Flüssigkeit 1 geöffnet und gegen die elastische Kraft des gummielastischen Körpers 6 geweitet werden.

Wenn der Austrag der ersten Flüssigkeit 1 und der zweiten Flüssigkeit 2 unterbrochen wird, weil kein Druck mehr auf die Austragskolben 32 ausgeübt wird, werden sich die Nebenleitungen 7 und gegebenenfalls auch die Hauptleitung 5 aufgrund der in dem gummielastischen Körper 6 gespeicherten elastischen Kraft wieder schließen. Dabei werden auch die Einmündungen 9 in die Hauptleitung 5 verschlossen. Die erste Flüssigkeit 1, die zweite Flüssigkeit 2 und das Flüssigkeitsgemisch 3 werden dabei aus der Hauptleitung 5 und den Nebenleitungen 7 in das offene Rohr 4 gedrückt. In dem Rohr 4 und gegebenenfalls in der Hauptleitung 5 kann das Flüssigkeitsgemisch 3 aushärten und einen Pfropfen bilden. Der Pfropfen kann zu einem späteren Zeitpunkt hydraulisch durch einen Druck auf die erste Flüssigkeit 1 ausgestoßen werden und die Vorrichtung ist sofort wieder einsatzbereit, um neues nicht ausgehärtetes Flüssigkeitsgemisch 3 zu produzieren.

In den Figuren 16 bis 22 sind Abbildungen eines zweiten Ausführungsbeispiels einer alternativen erfindungsgemäßen Vorrichtung zum Mischen der Flüssigkeiten und Teile davon in verschiedenen Ansichten gezeigt.

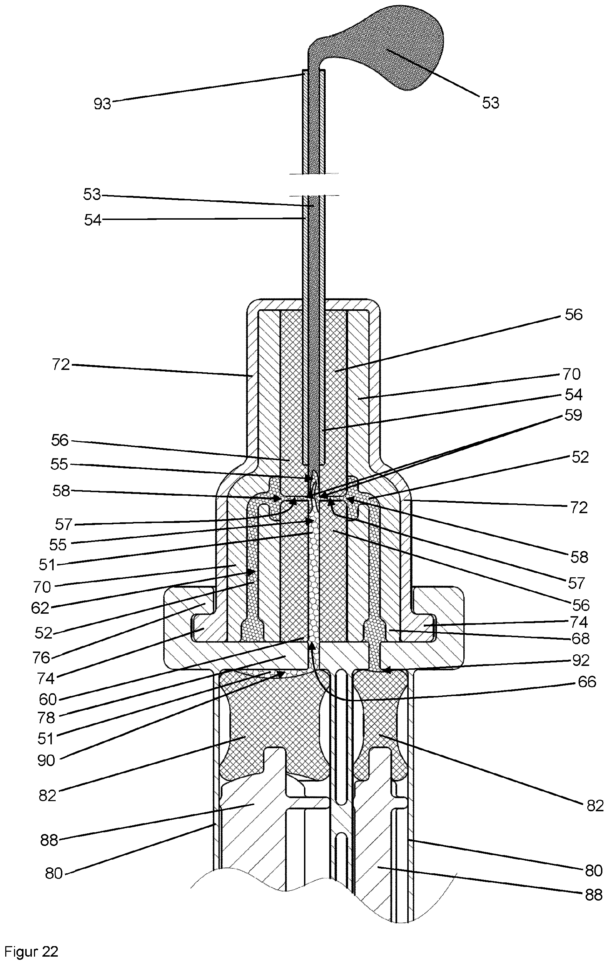

Die zweite alternative erfindungsgemäße Vorrichtung nach den Figuren 16 bis 22 ist dazu vorgesehen, eine erste Flüssigkeit 51 als Hauptkomponente (in Figur 22 durch eine Wabenstruktur mit großen Waben dargestellt) und eine zweite Flüssigkeit 52 als Nebenkomponente (in Figur 22 durch eine Wabenstruktur mit mittelgroßen Waben dargestellt) miteinander zu mischen. Dabei entsteht ein Flüssigkeitsgemisch 53 (in Figur 22 durch eine Wabenstruktur mit kleinen Waben dargestellt), das durch ein Rohr 54 ausgetragen werden kann, so wie das in Figur 22 gezeigt ist. Das Rohr 54 besteht vorzugsweise aus einem Kunststoff, der sich nicht mit dem Flüssigkeitsgemisch 53 verbindet, auch dann nicht, wenn das Flüssigkeitsgemisch 53 in dem Rohr 54 aushärtet.

Das Rohr 54 kann selbst gerade sein und kann in gerader Linie mit einer Hauptleitung 55 verbunden sein, der in einem gummielastischen Körper 56 ausgeformt ist. Die Hauptleitung 55 selbst ist vorzugsweise ebenfalls gerade, um einen Ausstoß von ausgehärtetem Flüssigkeitsgemisch 53 aus der Hauptleitung 55 und dem Rohr 54 zu erleichtern. Der gummielastische Körper 56 kann aus einem Elastomer bestehen oder aufgebaut werden. Geeignete Elastomere sind beispielsweise EPDM, Silikonkautschuk, Naturkautschuk, Butadien-Acrylnitril-Kautschuk und synthetischer Polyisoprenkautschuk. Der gummielastische Körper 56 kann als Hülse ausgeführt sein, wobei vorzugsweise in dem Inneren der Hülse die Hauptleitung 55 als axialer Kanal geführt sein kann.

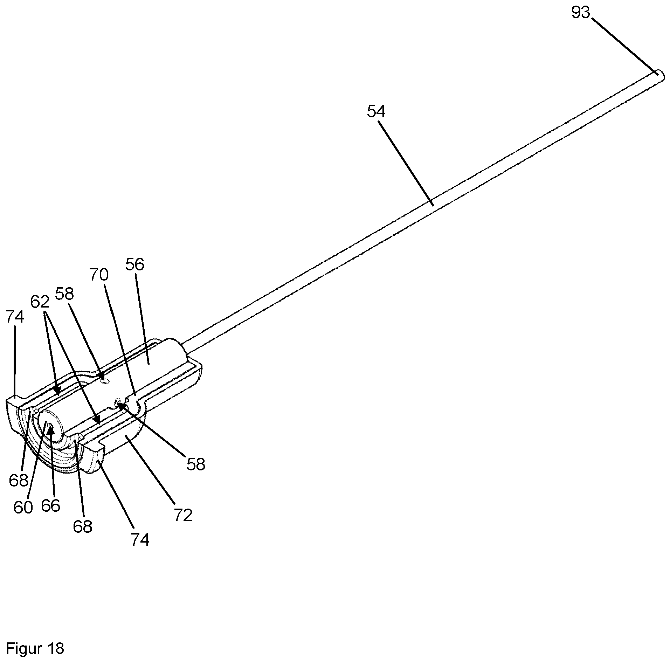

In dem gummielastischen Körper 56 können zusätzlich vier Nebenleitungen 57 ausgeformt sein, die sich in gerader Linie radial von der Hauptleitung 55 weg bis zu einer Oberfläche des gummielastischen Körpers 56 erstrecken. Hierzu können in einer Mantelfläche des hülsenförmigen gummielastischen Körpers 56 vier Nebenöffnungen 58 angeordnet sein, die in die Nebenleitungen 57 und damit in den gummielastischen Körper 56 hineinführen. Die Nebenöffnungen 58 können konisch geformt oder auf andere Weise geweitet sein, um ein Einpressen der zweiten Flüssigkeit 52 in die Nebenleitungen 57 zu erleichtern, wenn die Nebenleitungen 57 geschlossen sind (siehe Figuren 18 und 22).

Die Nebenleitungen 57 münden über Einmündungen 59 in die Hauptleitung 55. Die Nebenleitungen 57 können durch ein Durchstechen des gummielastischen Körpers 56 ausgeformt werden. Wesentlich ist, dass die Nebenleitungen 57 im entspannten Zustand des gummielastischen Körpers 56, also ohne dass eine Kraft beziehungsweise ein Druck auf die Leitungswandungen der Nebenleitungen 57 wirkt, verschlossen sind. In gleicher Weise kann vorgesehen sein, dass auch die Hauptleitung 55 im entspannten, also druckfreien Zustand verschlossen ist. Es kann aber auch vorgesehen sein, dass die Hauptleitung 55 nicht verschließbar ist.

Die Einmündungen 59 können alle an einem Ort in die Hauptleitung 55 münden. Hierdurch treffen Flüssigkeitsstrahlen der zweiten Flüssigkeit 52, die durch die Einmündungen 59 in die Hauptleitung 55 eingespritzt werden, aufeinander und die kinetische Energie der Flüssigkeitsstrahlen kann so zur Durchmischung der zweiten Flüssigkeit 52 mit der ersten Flüssigkeit 51 genutzt werden.

Das Rohr 54 kann in die Hauptleitung 55 eingesteckt sein, wobei das Rohr 54 nur so tief in die Hauptleitung 55 eingesteckt ist, dass es keine der Einmündungen 59 überdeckt. Theoretisch könnten auch Durchbrechungen in der Wandung des Rohrs 54 angeordnet sein, durch die die Nebenleitungen 57 über die Einmündungen 59 in die Hauptleitung münden.

Die erste Flüssigkeit 51 kann über einen Hauptanschluss 60 in die Hauptleitung 55 eingepresst werden. Der Hauptanschluss 60 bildet bei dieser Ausführung das zum Rohr 54 gegenüberliegende Ende der Hauptleitung 55. Zum Zuführen der zweiten Flüssigkeit 52 zu den Nebenleitungen 57 können mehrere Zuleitungen 62 vorgesehen sein. Die Zuleitungen 62 können über einen die Nebenöffnungen 58 umschließenden ringförmigen Kanal mit den Nebenleitungen 57 flüssigkeitsleitend und druckdicht verbunden sein, um sicherzustellen, dass die zweite Flüssigkeit 52 durch alle Nebenleitungen 57 in die Hauptleitung 55 eingespritzt werden kann, unabhängig davon über welche der Zuleitungen 62 die zweite Flüssigkeit 52 eingepresst wird. Alternativ können die Zuleitungen 62 auch auf andere Weise miteinander verbunden sein, um sicherzustellen, dass die zweite Flüssigkeit 52 in jede der Nebenöffnungen 58 in den gummielastischen Körper 56 eingepresst werden können.

Eine Hauptöffnung 66 kann am Hauptanschluss 60 in die Hauptleitung 55 münden. Die Hauptleitung 66 kann konisch geformt oder auf andere Weise geweitet sein, um ein Einpressen der ersten Flüssigkeit 51 in die Hauptleitung 55 zu erleichtern, wenn sie geschlossen ist.

Die Zuleitungen 62 können an dem den Einmündungen 58 gegenüberliegenden Enden mit jeweils einem Nebenanschluss 68 verbunden sein, über den die zweite Flüssigkeit 52 in die Zuleitungen 62 eingepresst werden kann. Der Hauptanschluss 60 und der Nebenanschluss 68 können in einer ebenen Dichtungsfläche als Flansch ausgeführt werden. Der Flansch kann auf einem passenden Gegenflansch einer Zweikomponenten-Kartusche befestigt sein oder werden.

Der Hauptanschluss 60 und der Nebenanschluss 68 können einteilig mit einem Innenteil 70 ausgeführt sein. Das Innenteil 70 kann aus einem Kunststoff bestehen. Die Zuleitungen 62 können im Inneren des Innenteils 70 ausgeformt sein. Zudem kann das Innenteil 70 eine Ausnehmung in Form einer zentralen durchgehenden Durchführung zur Aufnahme des gummielastischen Körpers 56 aufweisen, in der der gummielastische Körper 56 eingebaut ist. Das Innenteil 70 kann außen rotationssymmetrisch geformt sein. Hierdurch kann das Innenteil 70 gegen ein Gehäuse 72, das das Innenteil 70 umschließt, gedreht werden. Das Gehäuse 72 kann an seiner zur Zweikomponenten-Kartusche weisenden Seite Vorsprünge 74 aufweisen, die mit einer übergreifenden Aufnahme 76 an der Zweikomponenten-Kartusche nach Art eines Bajonett-Verschlusses verbunden werden können. Die Zweikomponenten-Kartusche kann Teil der erfindungsgemäßen Vorrichtung sein.

Die Zweikomponenten-Kartusche kann an ihrem vorderen Ende, das zum Verbinden an dem Flansch des Innenteils 70 mit dem gummielastischen Körper 56, zu Verbindung des Hauptanschlusses 60 und des Nebenanschlusses 68 vorgesehen ist, einen Kartuschenkopf 78 aufweisen. In dem Kartuschenkopf 78 können zwei Öffnungen angeordnet sein, die mit dem Hauptanschluss 60 und dem Nebenanschluss 68 zu verbinden sind. Die Zweikomponenten-Kartusche kann zwei parallel zueinander angeordnete Kartuschen 80 aufweisen. Die Zweikomponenten-Kartusche kann bis auf die beiden enthaltenen Flüssigkeiten 51, 52 aus Kunststoff bestehen. In den beiden Kartuschen 80 können axial verschiebbare Austragskolben 82 angeordnet sein, mit denen die erste Flüssigkeit 51 und die zweite Flüssigkeit 52 aus der Zweikomponenten-Kartusche heraus- und in die Hauptleitung 55 und in die Zuleitung 62 hineingepresst werden können. Die Kartusche 80 für die erste Flüssigkeit 51 kann einen größeren Innendurchmesser aufweisen als die Kartusche 80 für die zweite Flüssigkeit 52. Auf diese Weise kann ein Mischungsverhältnis erzeugt werden, bei dem ein geringerer Anteil der zweiten Flüssigkeit 52 einem größeren Volumen der ersten Flüssigkeit 51 beigemengt werden kann.

Die Austragskolben 82 können über Stößel 88 von der Rückseite der Zweikomponenten-Kartusche aus angetrieben werden. Die Kartuschen 80 weisen in ihrem Inneren zylindrische Innenräume auf, die ein Hauptreservoir 90 für die erste Flüssigkeit 51 und ein Nebenreservoir 92 für die zweite Flüssigkeit 52 begrenzen. Die erste Flüssigkeit 51 ist in dem Hauptreservoir 90 enthalten und die zweite Flüssigkeit 52 ist in dem Nebenreservoir 92 enthalten. Das Hauptreservoir 90 und das Nebenreservoir 92 sind an ihrer Vorderseite durch den Kartuschenkopf 78 und auf der Rückseite durch die Austragskolben 82 begrenzt, wobei der Inhalt mit den Austragskolben 82 durch die Öffnungen im Kartuschenkopf 78 in die Hauptleitung 55 und in die Zuleitung 62 gepresst werden kann.

Das fertige Flüssigkeitsgemisch 53 kann durch ein offenes Ende 93 des Rohrs 54 ausgetragen werden. Das Rohr 54 ist an dem, dem offenen Ende 93 gegenüberliegenden Ende mit der Hauptleitung 55 verbunden, beziehungsweise in die Hauptleitung 5 eingesteckt.

Die beiden Stößel 88 können an dem den Kartuschen 80 gegenüberliegenden Ende über ein Verbindungselement 94 miteinander verbunden sein. Hierdurch wird sichergestellt, dass die beiden Austragskolben 82 nur synchron vorgetrieben werden können, so dass immer das gleiche Mischungsverhältnis der ersten Flüssigkeit 51 und der zweiten Flüssigkeit 52 zum Mischen des Flüssigkeitsgemischs 53 erzeugt wird. Das Verbindungselement 94 kann auch als gemeinsames Griffstück zum manuellen Auspressen der ersten Flüssigkeit 51 und der zweiten Flüssigkeit 52 aus der Zweikomponenten-Kartusche nach Art einer Spritze genutzt werden und hierfür passend geformt sein. Hierfür kann auch an der Rückseite der Kartuschen 80 eine passende Gegenhalterung angeordnet sein.

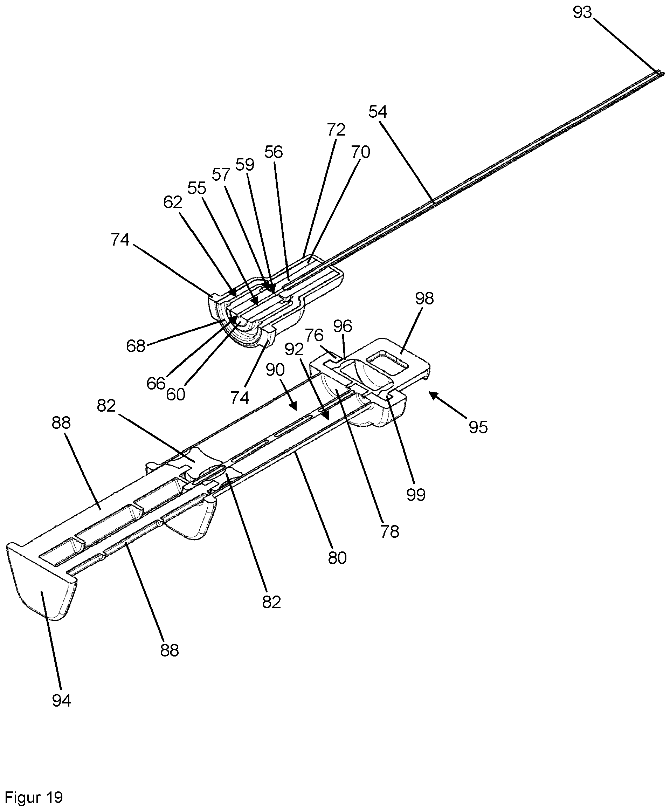

Die Zweikomponenten-Kartusche kann zur Lagerung mit einem Verschluss 95 verschlossen sein, der die beiden Öffnungen im Kartuschenkopf 78 verschließt und so die erste Flüssigkeit 51 und die zweite Flüssigkeit 52 in der Zweikomponenten-Kartusche einschließt (siehe Figuren 16 und 19). Der Verschluss 95 kann eine äußere Kappe 96, einen gegen die äußere Kappe 96 verdrehbar gelagerten Innenteil 97 und einen Griff 98 aufweisen. An dem Innenteil 97 können zwei Zapfen zum Verschließen der Öffnungen im Kartuschenkopf 78 der Zweikomponenten-Kartusche angeordnet sein. Die äußere Kappe 96 kann zwei Vorsprünge 99 aufweisen, so dass der Verschluss 95 mit der äußeren Kappe 96 analog dem Gehäuse 72 mit den Aufnahmen 76 an der Zweikomponenten-Kartusche nach Art eines Bajonett-Verschlusses verbunden werden kann.

Durch den Nebenanschluss 68 kann die zweite Flüssigkeit 52 durch die Zuleitung 62 in die Nebenöffnungen 58 gepresst werden, indem die zweite Flüssigkeit 52 mit dem Austragskolben 82 aus dem Nebenreservoir 92 in die Zuleitung 62 gepresst wird. Durch den Druck auf die zweite Flüssigkeit 52 weiten sich aufgrund einer elastischen Verformung des gummielastischen Körpers 56 die Nebenleitungen 57 und die Nebenleitungen 57 werden durchlässig für die zweite Flüssigkeit 52. Schließlich öffnen sich auch die Einmündungen 59 in die Hauptleitung 55 und die zweite Flüssigkeit 52 wird unter Druck in die in der Hauptleitung 55 geführte erste Flüssigkeit 51 eingespritzt. Die kinetische Energie, mit der die zweite Flüssigkeit 52 eingespritzt wird, und dadurch, dass die Flüssigkeitsströme der zweiten Flüssigkeit 52 aufeinandertreffen, wird eine starke Durchmischung der ersten Flüssigkeit 51 und der zweiten Flüssigkeit 52 zu dem Flüssigkeitsgemisch 53 bewirkt.

In gleicher Weise, wie die Nebenleitungen 57 durch einen Druck auf die zweite Flüssigkeit 52 geöffnet werden können, kann theoretisch auch die Hauptleitung 55 durch einen Druck auf die erste Flüssigkeit 51 geöffnet und gegen die elastische Kraft des gummielastischen Körpers 56 geweitet werden.

Wenn der Austrag der ersten Flüssigkeit 51 und der zweiten Flüssigkeit 52 unterbrochen wird, weil kein Druck mehr auf die Austragskolben 82 ausgeübt wird, werden sich die Nebenleitungen 57 aufgrund der in dem gummielastischen Körper 56 gespeicherten elastischen Kraft wieder schließen. Dabei werden auch die Einmündungen 59 in die Hauptleitung 55 verschlossen. Die erste Flüssigkeit 51, die zweite Flüssigkeit 52 und das Flüssigkeitsgemisch 53 werden dabei aus den Nebenleitungen 57 in das offene Rohr 54 gedrückt. In dem Rohr 54 und in der Hauptleitung 55 kann das Flüssigkeitsgemisch 53 aushärten und einen Pfropfen bilden. Der Pfropfen kann zu einem späteren Zeitpunkt hydraulisch durch einen Druck auf die erste Flüssigkeit 51 ausgestoßen werden und die Vorrichtung ist sofort wieder einsatzbereit, um neues nicht ausgehärtetes Flüssigkeitsgemisch 53 zu produzieren.

In den Figuren 23 und 24 sind Abbildungen eines dritten Ausführungsbeispiels einer weiteren alternativen erfindungsgemäßen Vorrichtung zum Mischen der Flüssigkeiten und Teile davon in verschiedenen Ansichten gezeigt.

Die dritte alternative erfindungsgemäße Vorrichtung nach den Figuren 23 und 24 ist dazu vorgesehen, eine erste Flüssigkeit und eine zweite Flüssigkeit miteinander zu mischen. Dabei entsteht ein Flüssigkeitsgemisch, das durch ein Rohr 104 ausgetragen werden kann. Das Rohr 104 besteht vorzugsweise aus einem Kunststoff, der sich nicht mit dem Flüssigkeitsgemisch verbindet, auch dann nicht, wenn das Flüssigkeitsgemisch in dem Rohr 104 aushärtet.

Das Rohr 104 kann selbst gerade sein und eine Hauptleitung 105 in seinem Inneren begrenzen. Vier gummielastische Körper 106 können an das Rohr 104 angelegt sein, die die Hauptleitung 105 im Bereich von Öffnungen in dem Rohr 104 begrenzen und zwar vorzugsweise derart, dass die Wandungen der Hauptleitung 105, die durch die gummielastischen Körper 106 geformt sind, mit der Innenwand des Rohrs 104 fluchten. Die Hauptleitung 105 selbst ist vorzugsweise gerade, um einen Ausstoß von ausgehärtetem Flüssigkeitsgemisch aus der Hauptleitung 105 und dem Rohr 104 zu erleichtern. Die gummielastischen Körper 106 können aus einem Elastomer bestehen oder aufgebaut werden. Geeignete Elastomere sind beispielsweise EPDM, Silikonkautschuk, Naturkautschuk, Butadien-Acrylnitril-Kautschuk und synthetischer Polyisoprenkautschuk. Die gummielastischen Körper 106 können als Hülse ausgeführt sein, wobei vorzugsweise in dem Inneren der Hülse jeweils eine Nebenleitung 107 als axialer Kanal geführt sein kann.

In jedem der gummielastischen Körper 106 kann jeweils eine Nebenleitungen 107 ausgeformt sein, die sich in gerader Linie radial von der Hauptleitung 105 weg bis zu einer Oberfläche der gummielastischen Körper 106 erstrecken. Hierzu können in einer Grundfläche der hülsenförmigen gummielastischen Körper 106 jeweils eine Nebenöffnung 108 angeordnet sein, die in die Nebenleitungen 107 und damit in die gummielastischen Körper 106 hineinführt. Die Nebenöffnungen 108 können geweitet sein, um ein Einpressen der zweiten Flüssigkeit in die Nebenleitungen 107 zu erleichtern, wenn die Nebenleitungen 107 geschlossen sind (siehe Figur 23).

Die Nebenleitungen 107 münden über Einmündungen 109 in die Hauptleitung 105. Die Einmündungen 109 sind in den Oberflächen der gummielastischen Körper 106 angeordnet, die die Hauptleitung 105 begrenzen. Die Nebenleitungen 107 können durch ein axiales Durchstechen der gummielastischen Körper 106 ausgeformt werden. Wesentlich ist, dass die Nebenleitungen 107 im entspannten Zustand der gummielastischen Körper 106, also ohne dass eine Kraft beziehungsweise ein Druck auf die Leitungswandungen der Nebenleitungen 107 wirkt, verschlossen sind.

Die Einmündungen 109 können alle an einem Ort in die Hauptleitung 105 münden. Hierdurch treffen Flüssigkeitsstrahlen der zweiten Flüssigkeit, die durch die Einmündungen 109 in die Hauptleitung 105 eingespritzt werden, aufeinander und die kinetische Energie der Flüssigkeitsstrahlen kann so zur Durchmischung der zweiten Flüssigkeit mit der ersten Flüssigkeit genutzt werden. Die Öffnungen im Rohr 104 sind so angeordnet, dass sie Einmündungen 109 offen lassen.

Die erste Flüssigkeit kann über einen Hauptanschluss 110 in die Hauptleitung 105 eingepresst werden. Zum Zuführen der zweiten Flüssigkeit zu den Nebenleitungen 107 können mehrere Zuleitungen 112 vorgesehen sein. Die Zuleitungen 112 können den Nebenleitungen 107 flüssigkeitsleitend und druckdicht verbunden sein, um sicherzustellen, dass die zweite Flüssigkeit in die Hauptleitung 105 eingespritzt werden kann. Die Zuleitungen 112 können miteinander verbunden sein, um sicherzustellen, dass die zweite Flüssigkeit in jede der Nebenöffnungen 108 in die gummielastischen Körper 106 eingepresst werden können. Die Zuleitungen 112 können aus einem festen Kunststoff bestehen. Zudem können die Zuleitungen 112 in die Nebenleitungen 107 eingesteckt sein, um die Nebenleitungen 107 im Bereich der Nebenöffnungen 108 zu weiten und eine dichte Verbindung herzustellen.

Die Zuleitungen 112 können an dem den Einmündungen 108 gegenüberliegenden Enden mit jeweils einem Nebenanschluss verbunden sein, über den die zweite Flüssigkeit in die Zuleitungen 112 eingepresst werden kann. Der Hauptanschluss 110 und der Nebenanschluss können mit einer Zweikomponenten-Kartusche (nicht gezeigt aber analog dem ersten oder zweiten Ausführungsbeispiel) befestigt sein oder werden.

Das fertige Flüssigkeitsgemisch kann durch ein offenes Ende 143 des Rohrs 104 ausgetragen werden. Der distale Teil des Rohrs 104 mit dem offenen Ende 143 ist mit dem proximalen Teil des Rohrs 104, das die Hauptleitung 105 im Wesentlichen begrenzt, verbunden. Anders ausgedrückt ist bei dieser Ausführung die Hauptleitung 105 im Wesentlichen durch ein (proximales) Rohr 104 begrenzt, das flüssigkeitsleitend und druckdicht mit dem (distalen Teil) Rohr 104 verbunden ist und einteilig mit dem (distalen Teil) Rohr 104 ausgeführt ist. Daher ist auch bei dieser Ausführung die Hauptleitung 105 mit dem Rohr 104 (dem distalen Teil) auf der dem offenen Ende 143 gegenüberliegenden Seite flüssigkeitsdurchlässig verbunden.

Durch den Nebenanschluss kann die zweite Flüssigkeit durch die Zuleitung 112 in die Nebenöffnungen 108 gepresst werden. Durch den Druck auf die zweite Flüssigkeit weiten sich aufgrund einer elastischen Verformung der gummielastischen Körper 106 die Nebenleitungen 107 und die Nebenleitungen 107 werden durchlässig für die zweite Flüssigkeit. Schließlich öffnen sich auch die Einmündungen 109 in die Hauptleitung 105 (siehe Figur 24) und die zweite Flüssigkeit wird unter Druck in die in der Hauptleitung 105 geführte erste Flüssigkeit eingespritzt. Die kinetische Energie, mit der die zweite Flüssigkeit eingespritzt wird, und dadurch, dass die Flüssigkeitsströme der zweiten Flüssigkeit aufeinandertreffen, wird eine starke Durchmischung der ersten Flüssigkeit und der zweiten Flüssigkeit zu dem Flüssigkeitsgemisch bewirkt.

Wenn der Austrag der ersten Flüssigkeit und der zweiten Flüssigkeit unterbrochen wird, werden sich die Nebenleitungen 107 aufgrund der in den gummielastischen Körpern 106 gespeicherten elastischen Kraft wieder schließen. Dabei werden auch die Einmündungen 109 in die Hauptleitung 105 verschlossen. Die zweite Flüssigkeit und das Flüssigkeitsgemisch werden dabei aus den Nebenleitungen 107 in das offene Rohr 104 gedrückt. In dem Rohr 104 und in der Hauptleitung 105 kann das Flüssigkeitsgemisch aushärten und einen Pfropfen bilden. Der Pfropfen kann zu einem späteren Zeitpunkt hydraulisch durch einen Druck auf die erste Flüssigkeit aus der Hauptleitung 105 herausgedrückt werden und die Vorrichtung ist sofort wieder einsatzbereit, um neues nicht ausgehärtetes Flüssigkeitsgemisch zu produzieren.

### Bezugszeichenliste

- 1, 51: Erste Flüssigkeit
- 2, 52: Zweite Flüssigkeit
- 3,53: Flüssigkeitsgemisch
- 4, 54, 104: Rohr
- 5, 55, 105: Hauptleitung
- 6, 56, 106: Gummielastischer Körper
- 7, 57. 107: Nebenleitung
- 8, 58, 108: Nebenöffnung
- 9, 59, 109: Einmündung
- 10, 60, 110: Hauptanschluss
- 12,62, 112: Zuleitung
- 14: Hauptzuleitung
- 16, 66: Hauptöffnung
- 18, 68: Nebenanschluss
- 20, 70: Innenteil
- 22, 72: Gehäuse
- 24, 74: Vorsprung
- 26, 76: Aufnahme
- 28, 78: Kartuschenkopf
- 30, 80: Kartusche
- 32, 82: Austragskolben
- 34: Abstreiflippe
- 36: Dichtung
- 38, 88: Stößel
- 40, 90: Hauptreservoir
- 42, 92: Nebenreservoir
- 43, 93, 143: Offenes Ende des Rohrs
- 44, 94: Verbindungselement
- 45, 95: Verschluss
- 46, 96: Kappe
- 47, 97: Innenteil
- 48, 98: Griff
- 49, 99: Vorsprung

## Patentansprüche

1. Vorrichtung zum Vermischen kleiner Volumina einer ersten Flüssigkeit (1, 51) und einer zweiten Flüssigkeit (2, 52), die Vorrichtung aufweisend
ein Rohr (4, 54, 104) mit einer Leitungsquerschnittsfläche von maximal 2 mm² oder mit einem Innendurchmesser von maximal 1,5 mm, wobei das Rohr (4, 54, 104) ein offenes Ende (43, 93, 143) hat,
eine Hauptleitung (5, 55, 105), die mit dem Rohr (4, 54, 104) auf der dem offenen Ende (43, 93, 143) gegenüberliegenden Seite flüssigkeitsdurchlässig verbunden ist,
zumindest einen Körper (6, 56, 106), der eine Leitungswandung der Hauptleitung (5, 55, 105) zumindest bereichsweise begrenzt oder der die gesamte Hauptleitung (5, 55, 105) begrenzt,
zumindest eine Nebenleitung (7, 57, 107), die in dem zumindest einen Körper (6, 56, 106) ausgeformt ist, wobei die zumindest eine Nebenleitung (7, 57, 107) sich von zumindest einer Nebenöffnung (8, 58, 108) in der Oberfläche des zumindest einen Körpers (6, 56, 106) bis zur Hauptleitung (5, 55, 105) erstreckt und über zumindest eine Einmündung (9, 59, 109) in dem zumindest einen Körper (6, 56, 106) in die Hauptleitung (5, 55, 105) mündet,
einen Hauptanschluss (10, 60, 110) zum Einleiten einer ersten Flüssigkeit (1, 51) in die Hauptleitung (5, 55, 105),
zumindest eine Zuleitung (12, 62, 112) zum Einleiten einer zweiten Flüssigkeit (2, 52) in die zumindest eine Nebenleitung (7, 57, 107), wobei die zumindest eine Zuleitung (12, 62, 112) mit der zumindest einen Nebenöffnung (8, 58, 108) flüssigkeitsdurchlässig verbunden ist,
**dadurch gekennzeichnet, dass** der zumindest eine Körper (6, 56, 106) zumindest ein gummielastischer Körper (6, 56, 106) ist, und dass
die zumindest eine Nebenleitung (7, 57, 107) in dem zumindest einen gummielastischen Körper (6, 56, 106) ohne Einwirken einer Kraft verschlossen ist und durch Ausüben eines Drucks auf eine der zumindest einen Nebenleitung (7, 57, 107) zugeführte zweite Flüssigkeit (2, 52) hydraulisch durch eine elastische Verformung des zumindest einen gummielastischen Körpers (6, 56, 106) zu öffnen ist und wobei sich die offene zumindest eine Nebenleitung (7, 57, 107) ohne Ausüben eines Drucks auf die zugeführte zweite Flüssigkeit (2, 52) durch die Rückstellkraft des elastisch verformten zumindest einen gummielastischen Körpers (6, 56, 106) verschließt.

2. Vorrichtung nach Anspruch 1, **dadurch gekennzeichnet, dass**
der zumindest eine gummielastische Körper (6, 56, 106) ein einzelner gummielastischer Körper (6, 56) ist, in dem die Hauptleitung (5, 55) und die zumindest eine Nebenleitung (7, 57) ausgeformt sind.

3. Vorrichtung nach Anspruch 2, **dadurch gekennzeichnet, dass**
das Rohr (4, 54) in die Hauptleitung (5, 55) eingesteckt ist, wobei bevorzugt das Rohr (4, 54) die zumindest eine Einmündung (9, 59) der zumindest einen Nebenleitung (7, 57) in die Hauptleitung (5, 55) nicht abdeckt.

4. Vorrichtung nach Anspruch 2 oder 3, **dadurch gekennzeichnet, dass**
der gummielastische Körper (6, 56) zumindest im Bereich der Nebenöffnung (8, 58) von einem Mantel abgedeckt wird, wobei bevorzugt der Mantel den gummielastischen Körper (6, 56) koaxial umschließt und die Hauptleitung (5, 55) entlang oder parallel zur Achse des Mantels in dem gummielastischen Körper (6, 56) verläuft, wobei besonders die zumindest eine Zuleitung als zumindest ein flüssigkeitsdurchlässiger Zwischenraum zwischen dem Mantel und dem gummielastischen Körper ausgeführt ist.

5. Vorrichtung nach einem der vorangehenden Ansprüche, **dadurch gekennzeichnet, dass**
das Rohr (4, 54, 104) flüssigkeitsdicht und druckdicht mit der Hauptleitung (5, 55, 105) verbunden ist, der Hauptanschluss (10, 60, 110) flüssigkeitsdicht und vorzugsweise auch druckdicht mit der Hauptleitung (5, 55, 105) verbunden ist und die zweite Zuleitung (12, 62, 112) flüssigkeitsdicht und vorzugsweise auch druckdicht mit der zumindest einen Nebenöffnung (8, 58, 108) verbunden ist, und/oder
der Innendurchmesser der Hauptleitung (5, 55, 105) oder der Innendurchmesser der Hauptleitung (5, 55, 105) in einem expandierten Zustand den gleichen Innenquerschnitt wie das Rohr (4, 54, 104) hat, und/oder
das Rohr (4, 54, 104) in seinem Inneren eine gerade zylindrische Leitung begrenzt, wobei bevorzugt in dem Rohr (4, 54, 104) keine Vorsprünge oder Vertiefungen sind, besonders bevorzugt das Rohr (4, 54, 104) in seinem Inneren glatt ist.

6. Vorrichtung nach einem der vorangehenden Ansprüche, **dadurch gekennzeichnet, dass**
die zumindest eine Einmündung (9, 59, 109) flächenbündig mit der Innenwand der Hauptleitung (5, 55, 105) ist und/oder das Rohr (4, 54, 104) flächenbündig mit der Hauptleitung (5, 55, 105) ist oder das Rohr (4, 54, 104) flächenbündig mit der Hauptleitung (5, 55, 105) im expandierten Zustand ist, und/oder
die zumindest eine Nebenleitung (7, 57, 107) wenigstens 1 mm lang ist, bevorzugt zumindest 5 mm lang ist, und/oder
die Hauptleitung (5, 55, 105) mindestens 3 mm lang ist, bevorzugt zumindest 5 mm lang ist.

7. Vorrichtung nach einem der vorangehenden Ansprüche, **dadurch gekennzeichnet, dass**
die Hauptleitung (5, 55, 105) über den Hauptanschluss (10, 60, 110) mit einem Hauptreservoir (40, 90) enthaltend die erste Flüssigkeit (1, 51) verbunden ist und die zumindest eine Zuleitung (12, 62, 112) mit einem Nebenreservoir (42, 92) enthaltend die zweite Flüssigkeit (2, 52) verbunden ist, wobei bevorzugt ein erster Austragskolben (32, 82) in dem Hauptreservoir (40, 90) angeordnet ist, mit dem die erste Flüssigkeit (1, 51) aus dem Hauptreservoir (40, 90) in die Hauptleitung (5, 55, 105) einpressbar ist, und in dem Nebenreservoir (42, 92) ein zweiter Austragskolben (32, 82) angeordnet ist, mit dem die zweite Flüssigkeit (2, 52) aus dem Nebenreservoir (42, 92) in die zumindest eine Zuleitung (12, 62, 112) einpressbar ist, wobei besonders bevorzugt der erste Austragskolben (32, 82) und der zweite Austragskolben (32, 82) miteinander fest verbunden sind,
wobei insbesondere die erste Flüssigkeit (1, 51) und die zweite Flüssigkeit (2, 52) Ausgangskomponenten eines medizinischen Gewebeklebstoffs sind und die Vorrichtung eine Vorrichtung zum Herstellen eines medizinischen Gewebeklebstoffs ist.

8. Vorrichtung nach einem der vorangehenden Ansprüche, **dadurch gekennzeichnet, dass**
das Rohr (4, 54, 104) eine Leitungsquerschnittsfläche von maximal 1 mm² oder einen Innendurchmesser von maximal 1 mm, bevorzugt von maximal 0,5 mm hat, und/oder die zumindest eine Einmündung (9, 59, 109) oder die zumindest eine Nebenleitung (7, 57, 107) im geöffneten Zustand einen Durchmesser von maximal 0,5 mm hat, bevorzugt einen Durchmesser von maximal 0,3 mm hat.

9. Vorrichtung nach einem der vorangehenden Ansprüche, **dadurch gekennzeichnet, dass**
die zumindest eine Nebenleitung (7, 57, 107) zumindest zwei Nebenleitungen (7, 57, 107) sind und die zumindest eine Einmündung (9, 59, 109) zumindest zwei Einmündungen (9, 59, 109) sind, wobei bevorzugt die zumindest zwei Einmündungen (9, 59, 109) auf gegenüberliegenden Seiten der Hauptleitung (5, 55, 105) angeordnet sind, so dass die durch die zumindest zwei Einmündungen (9, 59, 109) in die Hauptleitung (5, 55, 105) eingespritzte zweite Flüssigkeit (2, 52) in der Hauptleitung (5, 55, 105) direkt aufeinandertreffen, und/oder
die Hauptleitung (5, 55, 105) und die zumindest eine Zuleitung (12, 62, 112) mit einer Zweikomponenten-Kartusche verbunden oder verbindbar sind, wobei besonders bevorzugt die Hauptleitung (5, 55, 105) über den Hauptanschluss (10, 60, 110) mit einer ersten Kammer der Zweikomponenten-Kartusche verbunden oder verbindbar ist und die zumindest eine Zuleitung (12, 62, 112) mit einer zweiten Kammer der Zweikomponenten-Kartusche verbunden oder verbindbar ist, wobei ganz besonders bevorzugt in der ersten Kartusche die erste Flüssigkeit (1, 51) als erste Ausgangskomponente enthalten ist und in der zweiten Kartusche die zweite Flüssigkeit (2, 52) als zweite Ausgangskomponente enthalten ist.

10. Vorrichtung nach einem der vorangehenden Ansprüche, **dadurch gekennzeichnet, dass**
die Hauptleitung (5, 55) in dem zumindest einen gummielastischen Körper (6, 56) ausgeformt ist oder in einem weiteren gummielastischen Körper ausgeformt ist,
wobei die Hauptleitung (5, 55) in dem zumindest einen gummielastischen Körper (6, 56) oder in dem weiteren gummielastischen Körper ohne Einwirken einer Kraft verschlossen ist und durch Ausüben eines Drucks auf die der Hauptleitung (5, 55) zugeführte erste Flüssigkeit (1, 51) hydraulisch durch eine elastische Verformung des zumindest einen gummielastischen Körpers (6, 56) oder des weiteren gummielastischen Körpers zu öffnen ist und wobei sich die offene Hauptleitung (5, 55) ohne Ausüben eines Drucks auf die zugeführte erste Flüssigkeit (1, 51) durch die Rückstellkraft des elastisch verformten zumindest einen gummielastischen Körpers (6, 56) verschließt, und/oder
die zumindest eine Einmündung (9, 59, 109) in den zumindest einen gummielastischen Körper (6, 56, 106) trichterförmig ist und eine Öffnung in den zumindest einen gummielastischen Körper (6, 56, 106) bereitstellt, vorzugsweise auch dann, wenn kein Druck auf den zumindest einen gummielastischen Körper (6, 56, 106) wirkt.

11. Verfahren zur Herstellung einer Mischung zweier Flüssigkeiten, das Verfahren aufweisend die folgenden chronologischen Schritte:
A) Bereitstellen einer Zweikomponenten-Kartusche aufweisend ein Hauptreservoir (40, 90), in dem eine erste Flüssigkeit (1, 51) enthalten ist, und ein Nebenreservoir (42, 92), in dem eine zweite Flüssigkeit (2, 52) enthalten ist;
B) Auspressen der ersten Flüssigkeit (1, 51) aus dem Hauptreservoir (40, 90) in eine Hauptleitung (5, 55, 105) und Auspressen der zweiten Flüssigkeit (2, 52) aus dem Nebenreservoir (42, 92) in zumindest zwei geschlossene Nebenleitungen (7, 57, 107), wobei die zumindest zwei Nebenleitungen (7, 57, 107) in zumindest einem gummielastischen Körper (6, 56, 106) ausgeformt sind;
C) Öffnen der zumindest zwei Nebenleitungen (7, 57, 107) durch elastisches Verformen des zumindest einen gummielastischen Körpers (6, 56, 106) aufgrund einer von der zweiten Flüssigkeit (2, 52) ausgeübten und hydraulisch vermittelten Kraft auf die Wandungen der zumindest zwei Nebenleitungen (7, 57, 107);
D) Einspritzen der zweiten Flüssigkeit (2, 52) in die erste Flüssigkeit (1, 51) in der Hauptleitung (5, 55, 105) durch mehrere einander gegenüberliegende Einmündungen (9, 59, 109) der offenen zumindest zwei Nebenleitungen (7, 57, 107) in die Hauptleitung (5, 55, 105) und dadurch Vermischen der ersten Flüssigkeit (1, 51) mit der zweiten Flüssigkeit (2, 52), wobei die Ströme der eingespritzten zweiten Flüssigkeit (2, 52) in der Hauptleitung (5, 55, 105) aufeinandertreffen und dadurch das Vermischen der ersten Flüssigkeit (1, 51) mit der zweiten Flüssigkeit (2, 52) gefördert wird; und
E) Auspressen des Flüssigkeitsgemischs (3, 53) durch ein an die Hauptleitung (5, 55, 105) angeschlossenes Rohr (4, 54, 104).

12. Verfahren nach Anspruch 11, **dadurch gekennzeichnet, dass**
das Verfahren mit einer Vorrichtung nach einem der Ansprüche 1 bis 10 durchgeführt wird, wobei vorzugsweise
das Hauptreservoir (40, 90) mit dem Hauptanschluss (10, 60, 110) und der Hauptleitung (5, 55, 105) flüssigkeitsdurchlässig und druckdicht verbunden ist und das Nebenreservoir (42, 92) mit der zumindest einen Zuleitung (12, 62, 112) und den zumindest zwei Nebenleitungen (7, 57, 107) flüssigkeitsdurchlässig und druckdicht verbunden ist oder
nach Schritt A) und vor Schritt B) ein Schritt A2) erfolgt:
A2) Verbinden der Vorrichtung nach einem der Ansprüche 1 bis 10 oder der restlichen Vorrichtung nach einem der Ansprüche 1 bis 10 mit der Zweikomponenten-Kartusche, wobei das Hauptreservoir (40, 90) mit dem Hauptanschluss (10, 60, 110) und der Hauptleitung (5, 55, 105) flüssigkeitsdurchlässig und druckdicht verbunden wird und das Nebenreservoir (42, 92) mit der zumindest einen Zuleitung (12, 62, 112) und den zumindest zwei Nebenleitungen (7, 57, 107) flüssigkeitsdurchlässig und druckdicht verbunden wird.

13. Verfahren nach Anspruch 11 oder 12, **dadurch gekennzeichnet, dass**
das Auspressen in Schritt B) durch axiales Vortreiben je eines ersten Austragskolbens (32, 82) in dem Hauptreservoir (40, 90) und eines zweiten Austragskolbens (32, 82) in dem Nebenreservoir (42, 92) erfolgt, wobei bevorzugt der erste Austragskolben (32, 82) und der zweite Austragskolben (32, 82) synchron vorgetrieben werden und besonders bevorzugt der erste Austragskolben (32, 82) und der zweiten Austragskolben (32, 82) miteinander fest verbunden sind und in Schritt B) gemeinsam vorgetrieben werden.

14. Verfahren nach einem der Ansprüche 11 bis 13, **gekennzeichnet durch** die weiteren Schritte:
F) Unterbrechen des Auspressens der ersten Flüssigkeit (1, 51) und der zweiten Flüssigkeit (2, 52);
G) Verschließen der zumindest zwei Nebenleitungen (7, 57, 107) durch die von dem elastisch verformten zumindest einen gummielastischen Körper (6, 56, 106) wirkende Rückstellkraft;
H) Aushärten des Flüssigkeitsgemischs in dem Rohr (4, 54, 104) und, sofern dort enthalten, auch in der Hauptleitung (5, 55, 105) unter Bildung eines Pfropfens;
I) Erneutes Auspressen der ersten Flüssigkeit (1, 51) und der zweiten Flüssigkeit (2, 52);
J) Ausstoßen des Pfropfens durch das Rohr (4, 54, 104) aufgrund des hydraulischen Drucks der ersten Flüssigkeit (1, 51) in der Hauptleitung (5, 55, 105);
K) Öffnen der zumindest zwei Nebenleitungen (7, 57, 107) durch eine elastische Verformung des zumindest einen gummielastischen Körpers (6, 56, 106) aufgrund einer von der zweiten Flüssigkeit (2, 52) ausgeübten und hydraulisch vermittelten Kraft auf die Wandungen der zumindest zwei Nebenleitungen (7, 57, 107);
L) Einspritzen der zweiten Flüssigkeit (2, 52) in die erste Flüssigkeit (1, 51) in der Hauptleitung (5, 55, 105) durch die mehrere einander gegenüberliegenden Einmündungen (9, 59, 109) der offenen zumindest zwei Nebenleitungen (7, 57, 107) in die Hauptleitung (5, 55, 105) und dadurch Vermischen der ersten Flüssigkeit (1, 51) mit der zweiten Flüssigkeit (2, 52), wobei die Ströme der eingespritzten zweiten Flüssigkeit (2, 52) in der Hauptleitung (5, 55, 105) aufeinandertreffen und dadurch das Vermischen der ersten Flüssigkeit (1, 51) mit der zweiten Flüssigkeit (2, 52) gefördert wird; und
M) Auspressen des Flüssigkeitsgemischs (3, 53) durch das an die Hauptleitung (5, 55, 105) angeschlossene Rohr (4, 54, 104) und
die Hauptleitung (5, 55) in dem zumindest einen gummielastischen Körper (6, 56) ausgeformt ist oder in einem weiteren gummielastischen Körper ausgeformt ist wobei in Schritt C) ein Öffnen der Hauptleitung (5, 55) durch eine elastische Verformung des zumindest einen gummielastischen Körpers (6, 56) oder des weiteren gummielastischen Körpers aufgrund einer von der ersten Flüssigkeit (1, 51) ausgeübten und hydraulisch vermittelten Kraft auf die Wandungen der Hauptleitung (5, 55) erfolgt und
in Schritt D) das Einspritzen der zweiten Flüssigkeit (2, 52) in die erste Flüssigkeit (1, 51) in die geöffnete Hauptleitung (5, 55) durch zumindest eine Einmündung (9, 59) der offenen zumindest zwei Nebenleitungen (7, 57) in die geöffnete Hauptleitung (5, 55) erfolgt.

15. Verfahren nach Anspruch 14, **dadurch gekennzeichnet, dass**
in Schritt G) ein Verschließen der Hauptleitung (5, 55, 105) durch die von dem elastisch verformten zumindest einen gummielastischen Körper (6, 56, 106) wirkende Rückstellkraft oder durch die von dem elastisch verformten weiteren gummielastischen Körper (6, 56, 106) wirkende Rückstellkraft erfolgt,
in Schritt K) ein Öffnen der Hauptleitung (5, 55, 105) durch eine elastische Verformung des zumindest einen gummielastischen Körpers (6, 56, 106) oder des weiteren gummielastischen Körpers aufgrund einer von der ersten Flüssigkeit (1, 51) ausgeübten und hydraulisch vermittelten Kraft auf die Wandungen der Hauptleitung (5, 55, 105) erfolgt und
in Schritt L) das Einspritzen der zweiten Flüssigkeit (2, 52) in die erste Flüssigkeit (1, 51) in die geöffnete Hauptleitung (5, 55, 105) durch zumindest eine Einmündung (9, 59, 109) der offenen zumindest zwei Nebenleitungen (7, 57, 107) in die geöffnete Hauptleitung (5, 55, 105) erfolgt.

## Claims

1. A device for mixing small volumes of a first liquid (1, 51) and a second liquid (2, 52), the device comprising
a tube (4, 54, 104) having a line cross-sectional area of at most 2 mm² or having an inner diameter of at most 1.5 mm, wherein the tube (4, 54, 104) has an open end (43, 93, 143),
a main line (5, 55, 105), which is connected to the tube (4, 54, 104) in a liquid-permeable manner on the side opposite the open end (43, 93, 143),
at least one body (6, 56, 106), which delimits a line wall of the main line (5, 55, 105) at least in some regions or which delimits the entire main line (5, 55, 105),
at least one secondary line (7, 57, 107), which is formed in the at least one body (6, 56, 106), wherein the at least one secondary line (7, 57, 107) extends from at least one secondary opening (8, 58, 108) in the surface of the at least one body (6, 56, 106) up to the main line (5, 55, 105) and opens into the main line (5, 55, 105) via at least one mouth (9, 59, 109) in the at least one body (6, 56, 106),
a main connection (10, 60, 110) for introducing a first liquid (1, 51) into the main line (5, 55, 105),
at least one feed line (12, 62, 112) for introducing a second liquid (2, 52) into the at least one secondary line (7, 57, 107), wherein the at least one feed line (12, 62, 112) is connected to the at least one secondary opening (8, 58, 108) in a liquid-permeable manner,
**characterized in that** the at least one body (6, 56, 106) is at least one rubber-elastic body (6, 56, 106) and that
the at least one secondary line (7, 57, 107) is closed in the at least one rubber-elastic body (6, 56, 106) without the action of a force and is hydraulically openable by a pressure being exerted on a second liquid (2, 52), supplied to the at least one secondary line (7, 57, 107), by an elastic deformation of the at least one rubber-elastic body (6, 56, 106), and wherein the open at least one secondary line (7, 57, 107) closes without a pressure being exerted on the supplied second liquid (2, 52) by the restoring force of the elastically deformed at least one rubber-elastic body (6, 56, 106).

2. The device according to Claim 1, **characterized in that**
the at least one rubber-elastic body (6, 56, 106) is a single rubber-elastic body (6, 56) in which the main line (5, 55) and the at least one secondary line (7, 57) are formed.

3. The device according to Claim 2, **characterized in that**
the tube (4, 54) is inserted into the main line (5, 55), wherein preferably the tube (4, 54) does not cover the at least one mouth (9, 59) of the at least one secondary line (7, 57) into the main line (5, 55).

4. The device according to Claim 2 or 3, **characterized in that**
the rubber-elastic body (6, 56) is covered by a casing at least in the region of the secondary opening (8, 58), wherein preferably the casing coaxially surrounds the rubber-elastic body (6, 56), and the main line (5, 55) runs along or parallel to the axis of the casing in the rubber-elastic body (6, 56), wherein particularly preferably the at least one feed line is designed as at least one liquid-permeable intermediate space between the casing and the rubber-elastic body.

5. The device according to any one of the preceding claims, **characterized in that**
the tube (4, 54, 104) is connected to the main line (5, 55, 105) in a liquid-tight and pressure-tight manner, the main connection (10, 60, 110) is connected to the main line (5, 55, 105) in a liquid-tight manner and preferably also in a pressure-tight manner, and the second feed line (12, 62, 112) is connected to the at least one secondary opening (8, 58, 108) in a liquid-tight and preferably also pressure-tight manner, and/or
the inner diameter of the main line (5, 55, 105) or the inner diameter of the main line (5, 55, 105) in an expanded state has the same internal cross section as the tube (4, 54, 104), and/or
the tube (4, 54, 104) in the interior thereof delimits a straight cylindrical line, wherein preferably no projections or depressions are in the tube (4, 54, 104), particularly preferably the tube (4, 54, 104) is smooth in the interior thereof.

6. The device according to any one of the preceding claims, **characterized in that**
the at least one mouth (9, 59, 109) is flush with the inner wall of the main line (5, 55, 105) and/or the tube (4, 54, 104) is flush with the main line (5, 55, 105) or the tube (4, 54, 104) is flush with the main line (5, 55, 105) in the expanded state, and/or
the at least one secondary line (7, 57, 107) is at least 1 mm long, preferably at least 5 mm long, and/or
the main line (5, 55, 105) is at least 3 mm long, preferably at least 5 mm long.

7. The device according to any one of the preceding claims, **characterized in that**
the main line (5, 55, 105) is connected via the main connection (10, 60, 110) to a main reservoir (40, 90) containing the first liquid (1, 51), and the at least one feed line (12, 62, 112) is connected to a secondary reservoir (42, 92) containing the second liquid (2, 52), wherein preferably a first delivery plunger (32, 82) is arranged in the main reservoir (40, 90), by means of which first delivery plunger the first liquid (1, 51) is pressable from the main reservoir (40, 90) into the main line (5, 55, 105), and a second delivery plunger (32, 82) is arranged in the secondary reservoir (42, 92), by means of which second delivery plunger the second liquid (2, 52) is pressable from the secondary reservoir (42, 92) into the at least one feed line (12, 62, 112), wherein particularly preferably the first delivery plunger (32, 82) and the second delivery plunger (32, 82) are firmly connected to one another,
wherein in particular the first liquid (1, 51) and the second liquid (2, 52) are starting components of a medical tissue adhesive, and the device is a device for producing a medical tissue adhesive.

8. The device according to any one of the preceding claims, **characterized in that**
the tube (4, 54, 104) has a line cross-sectional area of at most 1 mm² or an inner diameter of at most 1 mm, preferably of at most 0.5 mm, and/or
the at least one mouth (9, 59, 109) or the at least one secondary line (7, 57, 107) in the opened state has a diameter of at most 0.5 mm, preferably a diameter of at most 0.3 mm.

9. The device according to any one of the preceding claims, **characterized in that**
the at least one secondary line (7, 57, 107) is at least two secondary lines (7, 57, 107) and the at least one mouth (9, 59, 109) is at least two mouths (9, 59, 109), wherein preferably the at least two mouths (9, 59, 109) are arranged on opposite sides of the main line (5, 55, 105), such that the second liquid (2, 52) injected into the main line (5, 55, 105) through the at least two mouths (9, 59, 109) directly meets in the main line (5, 55, 105), and/or
the main line (5, 55, 105) and the at least one feed line (12, 62, 112) are connected or connectable to a two-component cartridge, wherein preferably the main line (5, 55, 105) is connected or connectable via the main connection (10, 60, 110) to a first chamber of the two-component cartridge, and the at least one feed line (12, 62, 112) is connected or connectable to a second chamber of the two-component cartridge, wherein particularly preferably the first liquid (1, 51) is contained as the first starting component in the first cartridge and the second liquid (2, 52) is contained as a second starting component in the second cartridge.

10. The device according to any one of the preceding claims, **characterized in that** the main line (5, 55) is formed in the at least one rubber-elastic body (6, 56) or is formed in a further rubber-elastic body,
wherein the main line (5, 55) is closed in the at least one rubber-elastic body (6, 56) or in the further rubber-elastic body without the action of a force and is hydraulically openable by a pressure being exerted on the first liquid (1, 51) supplied to the main line (5, 55) by elastic deformation of the at least one rubber-elastic body (6, 56) or of the further rubber-elastic body, and wherein the open main line (5, 55) closes without a pressure being exerted on the supplied first liquid (1, 51) by the restoring force of the elastically deformed at least one rubber-elastic body (6, 56), and/or
the at least one mouth (9, 59, 109) into the at least one rubber-elastic body (6, 56, 106) is funnel-shaped and provides an opening into the at least one rubber-elastic body (6, 56, 106), preferably even when no pressure acts on the at least one rubber-elastic body (6, 56, 106).

11. A method for producing a mixture of two liquids, the method comprising the following chronological steps:
A) Providing a two-component cartridge having a main reservoir (40, 90), in which a first liquid (1, 51) is contained, and a secondary reservoir (42, 92), in which a second liquid (2, 52) is contained;
B) Pressing out the first liquid (1, 51) from the main reservoir (40, 90) into a main line (5, 55, 105) and pressing out the second liquid (2, 52) from the secondary reservoir (42, 92) into at least two closed secondary lines (7, 57, 107), wherein the at least two secondary lines (7, 57, 107) are formed in at least one rubber-elastic body (6, 56, 106);
C) Opening the at least two secondary lines (7, 57, 107) by elastic deformation of the at least one rubber-elastic body (6, 56, 106) on account of a force exerted by the second liquid (2, 52) and transmitted hydraulically to the walls of the at least one secondary line (7, 57, 107);
D) Injecting the second liquid (2, 52) into the first liquid (1, 51) in the main line (5, 55, 105) through a plurality of mutually opposite mouths (9, 59, 109) of the open at least two secondary lines (7, 57, 107) into the main line (5, 55, 105) and thereby mixing the first liquid (1, 51) with the second liquid (2, 52), wherein the flows of the injected second liquid (2, 52) meet in the main line (5, 55, 105) and, as a result, the mixing of the first liquid (1, 51) with the second liquid (2, 52) is promoted; and
E) Pressing out the liquid mixture (3, 53) through a tube (4, 54, 104) connected to the main line (5, 55, 105).

12. The method according to Claim 11, **characterized in that**
the method is carried out with a device according to any one of Claims 1 to 10, wherein preferably
the main reservoir (40, 90) is connected in a liquid-permeable and pressure-tight manner to the main connection (10, 60, 110) and the main line (5, 55, 105), and the secondary reservoir (42, 92) is connected to the at least one feed line (12, 62, 112) and the at least two secondary lines (7, 57, 107) in a liquid-permeable and pressure-tight manner, or
after step A) and before step B) a step A2) is carried out: A2) connecting the device according to any one of Claims 1 to 10 or the remaining device according to any one of Claims 1 to 10 to the two-component cartridge, wherein the main reservoir (40, 90) is connected to the main connection (10, 60, 110) and the main line (5, 55, 105) in a liquid-permeable and pressure-tight manner, and the secondary reservoir (42, 92) is connected to the at least one feed line (12, 62, 112) and the at least two secondary lines (7, 57, 107) in a liquid-permeable and pressure-tight manner.

13. The method according to any one of Claims 11 or 12, **characterized in that**
the pressing-out in step B) takes place by axially advancing a first delivery plunger (32, 82) in the main reservoir (40, 90) and a second delivery plunger (32, 82) in the secondary reservoir (42, 92), wherein preferably the first delivery plunger (32, 82) and the second delivery plunger (32, 82) are advanced in a synchronous manner, and particularly preferably the first delivery plunger (32, 82) and the second delivery plunger (32, 82) are firmly connected to one another and are advanced together in step B).

14. The method according to any one of Claims 11 to 13, **characterized by** the further steps:
F) Interrupting the pressing-out of the first liquid (1, 51) and of the second liquid (2, 52);
G) Closing the at least two secondary lines (7, 57, 107) by means of the restoring force acting from the elastically deformed at least one rubber-elastic body (6, 56, 106);
H) Curing the liquid mixture in the tube (4, 54, 104) and, if present, also in the main line (5, 55, 105) to form a plug;
I) Pressing out the first liquid (1, 51) and the second liquid (2, 52) again;
J) Expelling the plug through the tube (4, 54, 104) on account of the hydraulic pressure of the first liquid (1, 51) in the main line (5, 55, 105);
K) Opening the at least two secondary lines (7, 57, 107) by an elastic deformation of the at least one rubber-elastic body (6, 56, 106) on account of a force exerted by the second liquid (2, 52) and transmitted hydraulically to the walls of the at least two secondary lines (7, 57, 107);
L) Injecting the second liquid (2, 52) into the first liquid (1, 51) in the main line (5, 55, 105) through the plurality of mutually opposite mouths (9, 59, 109) of the open at least two secondary lines (7, 57, 107) into the main line (5, 55, 105) and thereby mixing the first liquid (1, 51) with the second liquid (2, 52), wherein the flows of the injected second liquid (2, 52) meet in the main line (5, 55, 105) and, as a result, the mixing of the first liquid (1, 51) with the second liquid (2, 52) is promoted; and
M) Pressing out the liquid mixture (3, 53) through the tube (4, 54, 104) connected to the main line (5, 55, 105), and
the main line (5, 55) is formed in the at least one rubber-elastic body (6, 56) or is formed in a further rubber-elastic body, wherein
in step C) the main line (5, 55) is opened by an elastic deformation of the at least one rubber-elastic body (6, 56) or of the further rubber-elastic body on account of a force exerted by the first liquid (1, 51) and transmitted hydraulically to the walls of the main line (5, 55), and
in step D) the second liquid (2, 52) is injected into the first liquid (1, 51) into the opened main line (5, 55) through at least one mouth (9, 59) of the open at least two secondary lines (7, 57) into the opened main line (5, 55).

15. The method according to claim 14, **characterized in that**
in step G) the main line (5, 55, 105) is closed by the restoring force acting from the elastically deformed at least one rubber-elastic body (6, 56, 106) or by the restoring force acting from the elastically deformed further rubber-elastic body (6, 56, 106),
in step K) the main line (5, 55, 105) is opened by an elastic deformation of the at least one rubber-elastic body (6, 56, 106) or of the further rubber-elastic body on account of a force exerted by the first liquid (1, 51) and hydraulically transmitted to the walls of the main line (5, 55, 105), and
in step L) the second liquid (2, 52) is injected into the first liquid (1, 51) into the opened main line (5, 55, 105) through at least one mouth (9, 59, 109) of the open at least two secondary lines (7, 57, 107) into the opened main line (5, 55, 105).

## Revendications

1. Dispositif pour le mélange de petits volumes d'un premier liquide (1, 51) et d'un second liquide (2, 52), le dispositif présentant
un tube (4, 54, 104) comportant une surface de section transversale de conduite d'au maximum 2 mm² ou comportant un diamètre intérieur d'au maximum 1,5 mm, le tube (4, 54, 104) ayant une extrémité ouverte (43, 93, 143),
une conduite principale (5, 55, 105) qui est reliée au tube (4, 54, 104) de manière perméable aux liquides sur le côté opposé à l'extrémité ouverte (43, 93, 143), au moins un corps (6, 56, 106) qui limite au moins dans certaines régions une paroi de conduite de la conduite principale (5, 55, 105) ou qui limite toute la conduite principale (5, 55, 105), au moins une conduite secondaire (7, 57, 107) qui est formée dans l'au moins un corps (6, 56, 106), l'au moins une conduite secondaire (7, 57, 107) s'étendant depuis au moins une ouverture secondaire (8, 58, 108) dans la surface de l'au moins un corps (6, 56, 106) jusqu'à la conduite principale (5, 55, 105) et débouchant dans la conduite principale (5, 55, 105) par l'intermédiaire d'au moins une embouchure (9, 59, 109) dans l'au moins un corps (6, 56, 106),
un raccord principal (10, 60, 110) pour l'introduction d'un premier liquide (1, 51) dans la conduite principale (5, 55, 105),
au moins une conduite d'alimentation (12, 62, 112) pour l'introduction d'un second liquide (2, 52) dans l'au moins une conduite secondaire (7, 57, 107), l'au moins une conduite d'alimentation (12, 62, 112) étant reliée de manière perméable aux liquides à l'au moins une ouverture secondaire (8, 58, 108),
**caractérisé en ce que** l'au moins un corps (6, 56, 106) est au moins un corps élastique en caoutchouc (6, 56, 106), et **en ce que**
l'au moins une conduite secondaire (7, 57, 107) est fermée dans l'au moins un corps élastique en caoutchouc (6, 56, 106) sans l'action d'une force et doit être ouverte hydrauliquement par une déformation élastique de l'au moins un corps élastique en caoutchouc (6, 56, 106) par l'application d'une pression sur un second liquide (2, 52) acheminé à l'au moins une conduite secondaire (7, 57, 107) et l'au moins une conduite secondaire (7, 57, 107) ouverte se fermant par la force de rappel de l'au moins un corps élastique en caoutchouc (6, 56, 106) déformé élastiquement sans l'application d'une pression sur le second liquide (2, 52) acheminé.

2. Dispositif selon la revendication 1, **caractérisé en ce que**
l'au moins un corps élastique en caoutchouc (6, 56, 106) est un seul corps élastique en caoutchouc (6, 56) dans lequel la conduite principale (5, 55) et l'au moins une conduite secondaire (7, 57) sont formées.

3. Dispositif selon la revendication 2, **caractérisé en ce que**
le tube (4, 54) est inséré dans la conduite principale (5, 55), de préférence le tube (4, 54) ne recouvrant pas l'au moins une embouchure (9, 59) de l'au moins une conduite secondaire (7, 57) dans la conduite principale (5, 55).

4. Dispositif selon la revendication 2 ou 3, **caractérisé en ce que**
le corps élastique en caoutchouc (6, 56) est recouvert par une enveloppe au moins dans la région de l'ouverture secondaire (8, 58), de préférence l'enveloppe entourant de manière coaxiale le corps élastique en caoutchouc (6, 56) et la conduite principale (5, 55) s'étendant le long de l'axe de l'enveloppe ou parallèlement à celui-ci dans le corps élastique en caoutchouc (6, 56), tout particulièrement l'au moins une conduite d'alimentation étant réalisée en tant qu'au moins un espace intermédiaire perméable aux liquides entre l'enveloppe et le corps élastique en caoutchouc.

5. Dispositif selon l'une des revendications précédentes, **caractérisé en ce que**
le tube (4, 54, 104) est relié de manière étanche aux liquides et de manière étanche à la pression à la conduite principale (5, 55, 105), le raccord principal (10, 60, 110) est relié de manière étanche aux liquides et de préférence aussi de manière étanche à la pression à la conduite principale (5, 55, 105) et la seconde conduite d'alimentation (12, 62, 112) est reliée de manière étanche aux liquides et de préférence aussi de manière étanche à la pression à l'au moins une ouverture secondaire (8, 58, 108), et/ou
le diamètre intérieur de la conduite principale (5, 55, 105) ou le diamètre intérieur de la conduite principale (5, 55, 105), dans un état expansé, a la même section transversale intérieure que le tube (4, 54, 104), et/ou
le tube (4, 54, 104) limite dans son intérieur une conduite cylindrique droite, de préférence aucune saillie ou cavité ne se trouvant dans le tube (4, 54, 104), de manière particulièrement préférée le tube (4, 54, 104) étant lisse dans son intérieur.

6. Dispositif selon l'une des revendications précédentes, **caractérisé en ce que**
l'au moins une embouchure (9, 59, 109) est à fleur de la paroi intérieure de la conduite principale (5, 55, 105) et/ou le tube (4, 54, 104) est à fleur de la conduite principale (5, 55, 105) ou le tube (4, 54, 104) est à fleur de la conduite principale (5, 55, 105) dans l'état expansé, et/ou
l'au moins une conduite secondaire (7, 57, 107) a une longueur d'au moins 1 mm, de préférence d'au moins 5 mm, et/ou
la conduite principale (5, 55, 105) a une longueur d'au moins 3 mm, de préférence d'au moins 5 mm.

7. Dispositif selon l'une des revendications précédentes, **caractérisé en ce que**
la conduite principale (5, 55 105) est reliée à un réservoir principal (40, 90) contenant le premier liquide (1, 51) par l'intermédiaire du raccord principal (10, 60, 110) et l'au moins une conduite d'alimentation (12, 62, 112) est reliée à un réservoir secondaire (42, 92) contenant le second liquide (2, 52), de préférence un premier piston de distribution (32, 82) étant disposé dans le réservoir principal (40, 90), avec lequel premier piston de distribution le premier liquide (1, 51) peut être pressé hors du réservoir principal (40, 90) et dans la conduite principale (5, 55, 105), et un second piston de distribution (32, 82) étant disposé dans le réservoir secondaire (42, 92), avec lequel second piston de distribution le second liquide (2, 52) peut être pressé hors du réservoir secondaire (42, 92) et dans l'au moins une conduite d'alimentation (12, 62, 112), de manière particulièrement préférée le premier piston de distribution (32, 82) et le second piston de distribution (32, 82) étant reliés solidement l'un à l'autre,
en particulier le premier liquide (1, 51) et le second liquide (2, 52) étant des composants de départ d'une colle tissulaire médicale et le dispositif étant un dispositif pour la production d'une colle tissulaire médicale.

8. Dispositif selon l'une des revendications précédentes, **caractérisé en ce que**
le tube (4, 54, 104) a une surface de section transversale de conduite d'au maximum 1 mm² ou un diamètre intérieur d'au maximum 1 mm, de préférence d'au maximum 0,5 mm, et/ou
l'au moins une embouchure (9, 59, 109) ou l'au moins une conduite secondaire (7, 57, 107) a, à l'état ouvert, un diamètre d'au maximum 0,5 mm, de préférence un diamètre d'au maximum 0,3 mm.

9. Dispositif selon l'une des revendications précédentes, **caractérisé en ce que**
l'au moins une conduite secondaire (7, 57, 107) est au moins deux conduites secondaires (7, 57, 107) et l'au moins une embouchure (9, 59, 109) est au moins deux embouchures (9, 59, 109), de préférence les au moins deux embouchures (9, 59, 109) étant disposées sur des côtés opposés de la conduite principale (5, 55, 105), de sorte que le second liquide (2, 52) injecté dans la conduite principale (5, 55, 105) à travers les au moins deux embouchures (9, 59, 109) se rencontre directement dans la conduite principale (5, 55, 105), et/ou
la conduite principale (5, 55, 105) et l'au moins une conduite d'alimentation (12, 62, 112) sont ou peuvent être reliées à une cartouche à deux composants, de manière particulièrement préférée la conduite principale (5, 55, 105) étant ou pouvant être reliée à une première chambre de la cartouche à deux composants par l'intermédiaire du raccord principal (10, 60, 110) et l'au moins une conduite d'alimentation (12, 62, 112) étant ou pouvant être reliée à une seconde chambre de la cartouche à deux composants, de manière tout particulièrement préférée le premier liquide (1, 51) étant contenu en tant que premier composant de départ dans la première cartouche et le second liquide (2, 52) étant contenu en tant que second composant de départ dans la seconde cartouche.

10. Dispositif selon l'une des revendications précédentes, **caractérisé en ce que** la conduite principale (5, 55) est formée dans l'au moins un corps élastique en caoutchouc (6, 56) ou est formée dans un autre corps élastique en caoutchouc, la conduite principale (5, 55) étant fermée dans l'au moins un corps élastique en caoutchouc (6, 56) ou dans l'autre corps élastique en caoutchouc sans l'action d'une force et devant être ouverte hydrauliquement par une déformation élastique de l'au moins un corps élastique en caoutchouc (6, 56) ou de l'autre corps élastique en caoutchouc par l'application d'une pression sur le premier liquide (1, 51) acheminé à la conduite principale (5, 55) et la conduite principale (5, 55) ouverte se fermant par la force de rappel de l'au moins un corps élastique en caoutchouc (6, 56) déformé élastiquement sans l'application d'une pression sur le premier liquide (1, 51) acheminé, et/ou l'au moins une embouchure (9, 59, 109) dans l'au moins un corps élastique en caoutchouc (6, 56, 106) est en forme d'entonnoir et fournit une ouverture dans l'au moins un corps élastique en caoutchouc (6, 56, 106), de préférence même lorsqu'aucune pression n'agit sur l'au moins un corps élastique en caoutchouc (6, 56, 106).

11. Procédé pour la production d'un mélange de deux liquides, le procédé présentant les étapes chronologiques suivantes :
A) fourniture d'une cartouche à deux composants présentant un réservoir principal (40, 90), dans lequel est contenu un premier liquide (1, 51), et un réservoir secondaire (42, 92), dans lequel est contenu un second liquide (2, 52) ;
B) expulsion par pression du premier liquide (1, 51) hors du réservoir principal (40, 90) dans une conduite principale (5, 55, 105) et expulsion par pression du second liquide (2, 52) hors du réservoir secondaire (42, 92) dans au moins deux conduites secondaires (7, 57, 107) fermées, les au moins deux conduites secondaires (7, 57, 107) étant formées dans au moins un corps élastique en caoutchouc (6, 56, 106) ;
C) ouverture des au moins deux conduites secondaires (7, 57, 107) par une déformation élastique de l'au moins un corps élastique en caoutchouc (6, 56, 106) en raison d'une force exercée par le second liquide (2, 52) et transmise hydrauliquement sur les parois des au moins deux conduites secondaires (7, 57, 107) ;
D) injection du second liquide (2, 52) dans le premier liquide (1, 51) dans la conduite principale (5, 55, 105) à travers plusieurs embouchures (9, 59, 109) opposées les unes aux autres des au moins deux conduites secondaires (7, 57, 107) ouvertes dans la conduite principale (5, 55, 105) et ainsi mélange du premier liquide (1, 51) avec le second liquide (2, 52), les écoulements du second liquide (2, 52) injecté se rencontrant dans la conduite principale (5, 55, 105) et le mélange du premier liquide (1, 51) avec le second liquide (2, 52) étant ainsi stimulé ; et
E) expulsion par pression du mélange de liquides (3, 53) à travers un tube (4, 54, 104) raccordé à la conduite principale (5, 55, 105).

12. Procédé selon la revendication 11, **caractérisé en ce que**
le procédé est exécuté avec un dispositif selon l'une des revendications 1 à 10, de préférence le réservoir principal (40, 90) étant relié de manière perméable aux liquides et de manière étanche à la pression au raccord principal (10, 60 110) et à la conduite principale (5, 55, 105) et le réservoir secondaire (42, 92) étant relié de manière perméable aux liquides et de manière étanche à la pression à l'au moins une conduite d'alimentation (12, 62, 112) et aux au moins deux conduites secondaires (7, 57, 107) ou après l'étape A) et avant l'étape B), une étape A2) est effectuée :
A2) liaison du dispositif selon l'une des revendications 1 à 10 ou du reste de dispositif selon l'une des revendications 1 à 10 à la cartouche à deux composants, le réservoir principal (40, 90) étant relié de manière perméable aux liquides et de manière étanche à la pression au raccord principal (10, 60 110) et à la conduite principale (5, 55, 105) et le réservoir secondaire (42, 92) étant relié de manière perméable aux liquides et de manière étanche à la pression à l'au moins une conduite d'alimentation (12, 62, 112) et aux au moins deux conduites secondaires (7, 57, 107).

13. Procédé selon la revendication 11 ou 12, **caractérisé en ce que**
l'expulsion par pression à l'étape B) est effectué par l'avancement axial de respectivement un premier piston de distribution (32, 82) dans le réservoir principal (40, 90) et d'un second piston de distribution (32, 82) dans le réservoir secondaire (42, 92), de préférence le premier piston de distribution (32, 82) et le second piston de distribution (32, 82) étant avancés de manière synchrone et de manière particulièrement préférée le premier piston de distribution (32, 82) et le second piston de distribution (32, 82) étant reliés solidement l'un à l'autre et avancés ensemble à l'étape B).

14. Procédé selon l'une des revendications 11 à 13, **caractérisé par** les autres étapes consistant à :
F) interrompre l'expulsion par pression du premier liquide (1, 51) et du second liquide (2, 52) ;
G) fermer les au moins deux conduites secondaires (7, 57, 107) par la force de rappel exercée par l'au moins un corps élastique en caoutchouc (6, 56, 106) déformé élastiquement ;
H) durcir le mélange de liquides dans le tube (4, 54, 104) et, dans la mesure où il y est contenu, aussi dans la conduite principale (5, 55, 105) en formant un bouchon ;
I) expulser par pression de nouveau le premier liquide (1, 51) et le second liquide (2, 52) ;
J) éjecter le bouchon à travers le tube (4, 54, 104) en raison de la pression hydraulique du premier liquide (1, 51) dans la conduite principale (5, 55, 105) ;
K) ouvrir les au moins deux conduites secondaires (7, 57, 107) par une déformation élastique de l'au moins un corps élastique en caoutchouc (6, 56, 106) en raison d'une force exercée par le second liquide (2, 52) et transmise hydrauliquement sur les parois des au moins deux conduites secondaires (7, 57, 107) ;
L) injecter le second liquide (2, 52) dans le premier liquide (1, 51) dans la conduite principale (5, 55, 105) à travers les embouchures (9, 59, 109) opposées les unes aux autres des au moins deux conduites secondaires (7, 57, 107) ouvertes dans la conduite principale (5, 55, 105) et ainsi mélanger le premier liquide (1, 51) avec le second liquide (2, 52), les écoulements du second liquide (2, 52) injecté se rencontrant dans la conduite principale (5, 55, 105) et le mélange du premier liquide (1, 51) avec le second liquide (2, 52) étant ainsi stimulé ; et
M) expulsion par pression du mélange de liquides (3, 53) à travers le tube (4, 54, 104) raccordé à la conduite principale (5, 55, 105) et
la conduite principale (5, 55) est formée dans l'au moins un corps élastique en caoutchouc (6, 56) ou est formée dans un autre corps élastique en caoutchouc, à l'étape C), une ouverture de la conduite principale (5, 55) étant effectuée par une déformation élastique de l'au moins un corps élastique en caoutchouc (6, 56) ou de l'autre corps élastique en caoutchouc en raison d'une force exercée par le premier liquide (1, 51) et transmise hydrauliquement sur les parois de la conduite principale (5, 55) et
à l'étape D), l'injection du second liquide (2, 52) dans le premier liquide (1, 51) dans la conduite principale (5, 55) ouverte étant effectuée à travers au moins une embouchure (9, 59) des au moins deux conduites secondaires (7, 57) ouvertes dans la conduite principale (5, 55) ouverte.

15. Procédé selon la revendication 14, **caractérisé en ce que**
à l'étape G), une fermeture de la conduite principale (5, 55, 105) est effectuée par la force de rappel exercée par l'au moins un corps élastique en caoutchouc (6, 56, 106) déformé élastiquement ou par la force de rappel exercée par l'autre corps élastique en caoutchouc (6, 56, 106) déformé élastiquement,
à l'étape K), une ouverture de la conduite principale (5, 55, 105) est effectuée par une déformation élastique de l'au moins un corps élastique en caoutchouc (6, 56, 106) ou de l'autre corps élastique en caoutchouc en raison d'une force exercée par le premier liquide (1, 51) et transmise hydrauliquement sur les parois de la conduite principale (5, 55, 105) et
à l'étape L), l'injection du second liquide (2, 52) dans le premier liquide (1, 51) dans la conduite principale (5, 55, 105) ouverte est effectuée à travers au moins une embouchure (9, 59, 109) des au moins deux conduites secondaires (7, 57, 107) ouvertes dans la conduite principale (5, 55, 105) ouverte.
